(19) Europäisches Patentamt
European Patent Office
Office européen des brevets

(11) Veröffentlichungsnummer: **0 049 448**
**B1**

# (12) EUROPÄISCHE PATENTSCHRIFT

(45) Veröffentlichungstag der Patentschrift: **24.08.88**

(51) Int. Cl.⁴: **C 07 D 501/20**, C 07 D 498/04, C 07 D 499/46, A 61 K 31/545 // C07D277/40

(21) Anmeldenummer: **81107679.3**

(22) Anmeldetag: **28.09.81**

(54) **Beta-Lactamantibiotika, Verfahren zu deren Herstellung sowie sie enthaltende Mittel.**

(30) Priorität: **08.10.80 DE 3037997**

(43) Veröffentlichungstag der Anmeldung:
**14.04.82 Patentblatt 82/15**

(45) Bekanntmachung des Hinweises auf die Patenterteilung:
**24.08.88 Patentblatt 88/34**

(84) Benannte Vertragsstaaten:
**AT BE CH DE FR GB IT LI NL SE**

(56) Entgegenhaltungen:
**EP - A - 0 004 956**
**US - A - 4 014 869**

**DERWENT JAPANESE PATENT REPORTS, vol.7, no.9, 08-04-1968**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Boberg, Michael, Dr., Bergerheide 101A, D-5600 Wuppertal 1 (DE)**
Erfinder: **Metzger, Karl Georg, Dr., Pahlkestrasse 15, D-5600 Wuppertal 1 (DE)**

**EP 0 049 448 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft β-Lactamverbindungen, Verfahren zu ihrer Herstellung sowie Mittel, wie Arzneimittel insbesondere antibakterielle Mittel, ferner Mittel zur Förderung des Wachstums und zur Verbesserung der Futterverwertung bei Tieren sowie Antioxidantien.

Es sind bereits β-Lactamverbindungen, die eine substituierte Acrylamidoseitenkette tragen, beschrieben worden.

So sind zum Beispiel in der BE-PS 633 397 und in der US-PS 3 622 569 Penicillinverbindungen erwähnt, die das Strukturelement

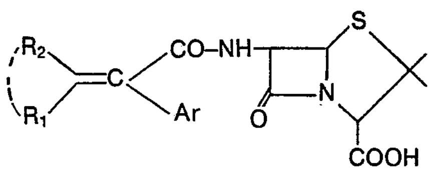

enthalten, worin Ar ein substituierter Phenylring oder ein Heterocyclus sein kann und $R^1$ und $R^2$ substituierte oder unsubstituierte Alkylgruppen oder eine Cycloalkylgruppe darstellen.

Weiterhin beschreibt die US-PS 4 014 869 Cefalosporinverbindungen mit einer Z-konfigurierten Acrylamidoseitenkette, die in 2- und 3-Stellung u.a. aromatische bzw. heterocyclische Reste trägt:

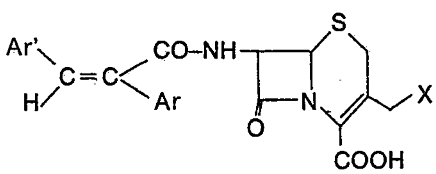

Die vorliegende Erfindung betrifft β-Lactamverbindungen, die der allgemeinen Formel I

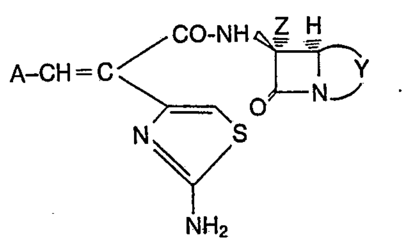 (I)

entsprechen, worin

A Wasserstoff, gegebenenfalls cyclisches, Alkyl, Alkenyl oder Alkinyl mit bis zu 18 C-Atomen, die durch O, S oder Methoxyimino substituiert sein können, einen Rest der Formel

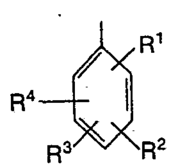

bedeutet,

worin $R^1$, $R^2$, $R^3$ und $R^4$ unabhängig voneinander Wasserstoff, Halogen, Alkyl mit bis zu 6 Kohlenstoffatomen, Aryl, eine Gruppe $-OCOR^5$, eine Gruppe 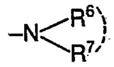,

Nitro, Cyano, Trifluormethyl, Hydroxy, Alkoxy oder Alkylthio mit bis zu 6 C-Atomen, Hydroxycarbonyl, Alkoxycarbonyl mit bis zu 6 C-Atomen im Alkylteil, Aminocarbonyloxy, Sulfonyl oder Sulfo bedeuten, worin wiederum Aryl einen substituierten oder unsubstituierten carbocyclischen aromatischen Ring oder 5- oder 6-gliedrigen heterocyclischen Ring bedeutet,

und worin $R^5$, gegebenenfalls substituiertes und gegebenenfalls cyclisches, Alkyl, Alkenyl oder Alkinyl mit bis zu 6 C-Atomen bedeutet,

und worin $R^6$ und $R^7$ unabhängig voneinander Wasserstoff, gegebenenfalls substituiertes und gegebenenfalls cyclisches, Alkyl, Alkenyl oder Alkinyl mit bis zu 6 C-Atomen oder Alkanoyl mit bis zu 6 C-Atomen oder gemeinsam Alkyl oder Alkanoyl mit bis zu 6 C-Atomen bedeuten,

oder Naphthyl, 5-Methylisoxazol-3-yl, Furyl, Thienyl oder Pyridinyl bedeutet,

und worin

Y in der Form der freien Säure einen Rest der Formel

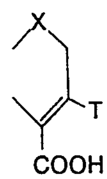

oder

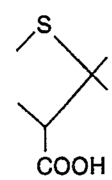

darstellt,

worin X Schwefel oder Sauerstoff bedeutet und T ein in der Cephalosporinchemie üblicher Rest ist,

und worin

Z H oder Methoxy bedeutet.

Die erfindungsgemässen Verbindungen haben sehr gute antibakterielle Eigenschaften.

Wenn A einen Alkylrest darstellt, dann bevorzugt einen geradkettigen oder verzweigten, gegebenenfalls substituierten Rest mit bis zu 12 C-Atomen und speziell mit bis zu 6 C-Atomen.

Hinsichtlich der Definition von Y sind bevorzugte Verbindungen solche, in denen

X Schwefel oder Sauerstoff bedeutet und

T Wasserstoff, $C_1$–$C_4$-Alkyl, Halogen, $C_1$–$C_4$-Alkoxi, Hydroximethyl, Formyloximethyl, $C_1$–$C_4$-Alkylcarbonyloximethyl, Aminocarbonyloximethyl, Pyridiniummethyl, 4-Carbamoylpyridiniummethyl oder Heterocyclylthiomethyl

bedeutet, wobei Heterocyclyl vorzugsweise für einen Rest der Formel

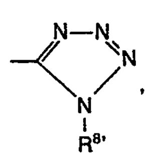, 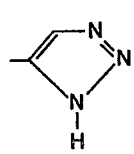, 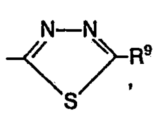, 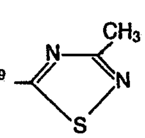

steht, worin

$R^8$ Wasserstoff, Methyl, 2-Dimethylaminoethyl, Carboximethyl oder Sulfomethyl und

$R^9$ Wasserstoff oder Methyl bedeuten.

In den Verbindungen der Formel I existiert zu jeder Strukturformel eine E- und eine Z-konfigurierte Verbindung gemäss der J. Amer. Chem. Soc. 90, 509 (1968) beschriebenen E/Z-Nomenklatur. Erfindungsgemäss bevorzugt sind solche in der Z-Konfiguration.

Die Verbindungen der Formel I können als freie Säuren, Ester, als innere Salze oder als nicht toxische, pharmazeutisch verträgliche Salze der sauren Carboxylgruppe, wie die Natrium-, Kalium-, Magnesium-, Calcium-, Aluminium- und Ammoniumsalze und nicht-toxische substituierte Ammoniumsalze, mit Aminen wie
Di- und Triniedrigalkylaminen, Procain,
Dibenzylamin, N,N'-Dibenzylethylendiamin,
N-Benzyl-β-phenylethylamin, N-Methyl- und
N-Ethylmorpholin, 1-Ephenamin,
Dehydroabietylamin,
N,N'-Bis-dehydroabiethylethylendiamin,
N-Niedrigalkylpiperidin
und andere Amine, die zur Bildung von Salzen von Penicillinen oder Cephalosporinen verwendet werden können, vorliegen.

Bevorzugte Verbindungen in der Z-Konfiguration sind solche

worin
Z Wasserstoff oder Methoxi,
X Schwefel
T einen Rest

$-CH_2OCOCH_3$, 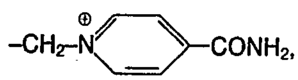, 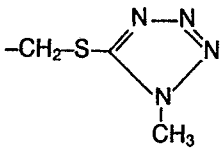

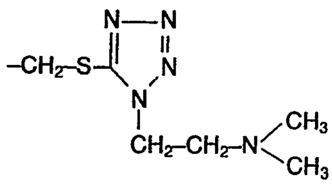 oder $-CH_2OCONH_2$

und
A einen Rest der Formel

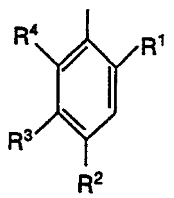, 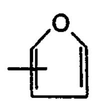, 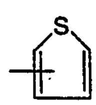, 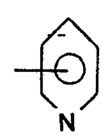, 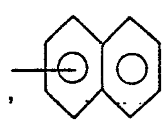

bedeuten.

Die jeweiligen Z- und E-Formen derselben Strukturformel sind in der Regel unterschiedlich wirksame Substanzen, die getrennt voneinander oder gemeinsam hergestellt werden können.

Die Verbindungen der allgemeinen Formel I können dadurch erhalten werden, dass Verbindungen der allgemeinen Formel II

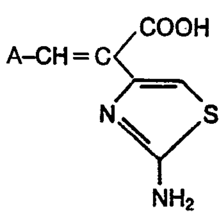 II

worin A die oben angegebene Bedeutung hat, in denen die Aminogruppe geschützt oder ungeschützt vorliegen kann, nach Aktivierung der Carboxylgruppe durch Überführung in ein gemischtes Anhydrid, beispielsweise mit Chlorameisensäureethylester oder -isobutylester, nach Überführung in das Säurehalogenid oder nach Überführung in einen aktivierten Ester mit beispielsweise N-Hydroxisuccinimid und Dicyclohexylcarbodiimid, mit Verbindungen der allgemeinen Formel III (die auch in der Mono- oder Disilylform oder in Form von spaltbaren Estern vorliegen können)

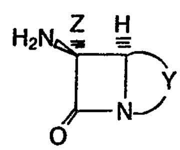 III,

worin Y und Z die oben genannte Bedeutung besitzen, zur Umsetzung gebracht werden und anschliessend gegebenenfalls Schutzgruppen entfernt werden.

Bevorzugt werden die Verbindungen der Formel II nach Aktivierung der Carboxylgruppe mit Phosphoroxichlorid, Phosphorpentachlorid oder als Ester mit einem Alkohol der Formel IV

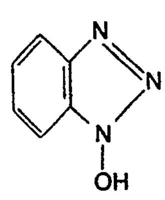 IV,

ohne vorherige Einführung einer Amino-Schutzgruppe, mit einer Verbindung der Formel III umgesetzt.

Die erfindungsgemässen Verbindungen der Formel I lassen sich auch erhalten durch Umsetzung von Verbindungen der Formel I, in denen T die Bedeutung von $-CH_2-OCO$-Niedrigalkyl, insbesondere $-CH_2-OCOCH_3$ hat, mit Nukleophilen, beispielsweise

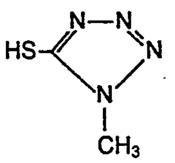

oder

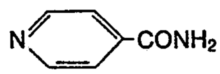

Verbindungen der Formel I erhält man weiterhin, wenn man Verbindungen der allgemeinen Formel I, in denen T die Bedeutung von $CH_2OH$ hat, mit $O=C=NSO_2Cl$ oder $O=C=N-COCCl_3$ umsetzt und anschliessend die $SO_2Cl$- oder $-COCCl_3$-Gruppe abspaltet.

Verbindungen der Formel I kann man auch erhalten, wenn man Verbindungen der Formel I, in denen Z Wasserstoff bedeutet und die in der Form der freien Säuren, der Salze oder spaltbarer Ester vorliegen, in Gegenwart eines Alkoholats, vorzugsweise Methanolats, beispielsweise $LiOCH_3$, mit einem Hypochlorit, beispielsweise $(CH_3)_3COCl$ umsetzt und gegebenenfalls Schutzgruppen entfernt.

Die zur Herstellung der erfindungsgemässen Verbindungen notwendigen, in der Literatur bisher nicht beschriebenen Verbindungen der Formel II lassen sich durch eine Reihe von Verfahren darstellen. Es hat sich gezeigt, dass die drei folgenden Verfahren am zweckmässigsten sind. Nach Verfahren (1) wird im ersten Schritt eine Verbindung der Formel V

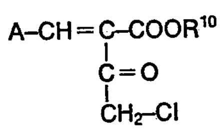

V,

in der A die oben genannte Bedeutung hat und $R^{10}$ $C_1-C_4$ Niedrigalkyl, vorzugsweise Ethyl, bedeutet, durch Kondensation einer Verbindung der Formel VI

$$Cl-CH_2-CO-CH_2-COOR^{10}, \quad VI$$

in der $R^{10}$ die oben genannte Bedeutung hat, mit einer Carbonylverbindung der Formel VII

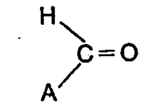

VII,

in der A die oben genannte Bedeutung hat, hergestellt. Für diese Reaktion sind in der Literatur eine Reihe von Verfahren beschrieben, es hat sich jedoch gezeigt, dass das in J. Amer. Chem. Soc. 66, 1933 (1944) an nur einem Beispiel erprobte Verfahren, welches das Katalysatorsystem Piperidin/Eisessig verwendet, zur Synthese einer grossen Zahl von Verbindungen der Formel V angewendet werden kann. Dies kann in unterschiedlichen Lösungsmitteln unter unter unterschiedlichen Bedingungen geschehen, (z.B. Benzol/Wasserabscheider/Rückfluss oder DMF/Trockenmittel, z.B. Molekularsieb/50°). Umsetzung einer Verbindung der Formel V mit Thioharnstoff in einem polaren Lösungsmittel wie beispielsweise Acetonitril, am besten jedoch in einem Hydroxylgruppen-haltigen Lösungsmittel wie beispielsweise Methanol oder Ethanol oder einem Lösungsmittel-Gemisch wie beispielsweise Ethanol/Wasser oder Tetrahydrofuran/Wasser in einem Temperaturbereich zwischen 0° und 100 °C, vorzugsweise zwischen 20° und 50 °C, gegebenenfalls unter Zusatz eines Puffers, wie beispielsweise Natriumacetat, führt zu einer Verbindung, die in der Form der freien Base der Formel VIII entspricht

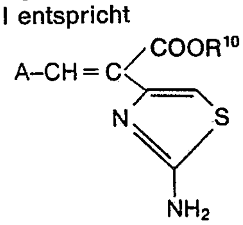

VIII,

in der A und $R^{10}$ die oben genannte Bedeutung haben. Eine Verbindung der Formel VIII kann, sowohl in Form der freien Base entsprechend Formel VIII, als auch in Form von deren Hydrochlorid isoliert werden. Sie lässt sich durch alkalische Esterverseifung mit beispielsweise 2 n NaOH in Methanol, Ethanol oder Dioxan als Lösungsmittel bei Temperaturen von 0° bis Rückflusstemperatur in eine Carbonsäure der Formel II überführen.

Nach der Verfahrenvariante (2) wird eine Verbindung der Formel VIII auf einem Wege erhalten, der sich vom Weg (1) prinzipiell nur durch die Reihenfolge der Schritte unterscheidet. Das Kondensationsprodukt IX aus Acetessigester und einer Carbonylverbindung der Formel VII reagiert mit Brom zu einem 1,4-Dibromid X

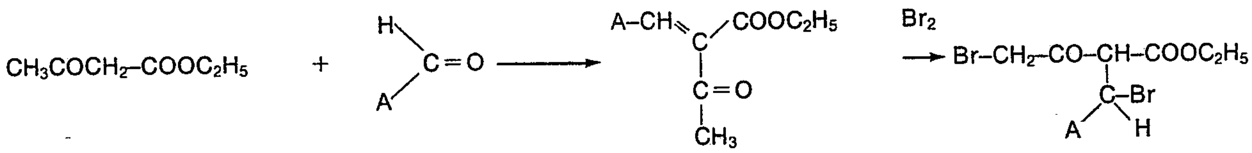


VII IX X,

in dem A die oben genannte Bedeutung hat. Die Reaktion verläuft am besten, wenn man sie bei −10° bis 0 °C in einem unpolaren Lösungsmittel wie beispielsweise Methylenchlorid, Chloroform oder Toluol durchführt. Eine Verbindung der Formel X lässt sich mit Thioharnstoff unter den schon

erwähnten Bedingungen unter gleichzeitiger Cyclisierung und HBr-Eliminierung zu einer Verbindung der Formel VIII umsetzen.

Nach einem weiteren Verfahren zur Herstellung der erfindungsgemässen Verbindungen wird die Verbindung der allgemeinen Formel XII,

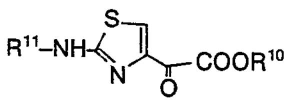

XII

in der $R^{10}$ die oben genannte Bedeutung hat und $R^{11}$ eine Amino-Schutzgruppe wie beispielsweise Formyl oder tert.-Butoxycarbonyl darstellt,
mit einem Phosphonat der allgemeinen Formel XIII,

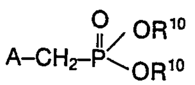

XIII

in der A und $R^{10}$ die oben genannten Bedeutungen haben nach Deprotonierung von XIII mit einer Base wie z.B. Natriumhydrid, Lithiumdiisopropylamid, Lithiumhydrid, Butyllithium oder Kalium-tert.-butanolat, in einem organischen Lösungsmittel wie z.B. Tetrahydrofuran zu einer Verbindung der allgemeinen Formel XIV

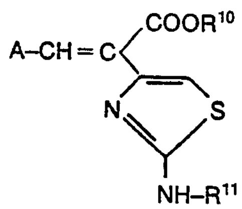

XIV

umgesetzt.

Aus dieser Verbindung XIV lässt sich durch alkalische Esterverseifung eine Carbonsäure der Formel XV,

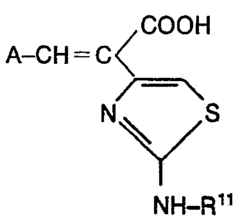

XV

gewinnen, die dann, wie für Verbindungen der Formel II beschrieben, aktiviert und mit Verbindungen der Formel III umgesetzt werden können, wodurch nach Abspaltung der Schutzgruppen eine Verbindung der Formel I erhalten wird.

Weiterhin kann der Substituent A auf einer der beschriebenen Synthese-Zwischenstufen variiert werden.

Werden die erfindungsgemässen Verbindungen als Gemisch der E- und Z-Isomeren hergestellt, so kann eine Trennung dieser Isomeren auf jeder der beschriebenen Synthese-Zwischenstufen V, VIII oder II mit Hilfe üblicher Trennmethoden wie z.B. Destillation, Kristallisation, Chromatografie sowie durch selektive chemische Umsetzung nur eines Isomeren in das Produkt der nächsten Synthesestufe.

Weiterhin ist es in einer Reihe von Fällen möglich, eine Isomerisierung, d.h. eine Überführung des einen Isomeren in das andere durch Behandlung mit Säuren oder mit Basen in einem polaren organischen oder wässrig-organischen oder wässrigen Lösungsmittel wie z.B. HCl in Ethanol oder verdünnter Natronlauge zu erreichen.

Die erfindungsgemässen Verbindungen zeigen ein breites antibakterielles Spektrum gegen grampositive und gram-negative Keime, insbesondere gegen Enterobakteriaceae, vor allem gegen solche mit β-Lactamasebildung.

Ferner verbessern die erfindungsgemässen Verbindungen das Wachstum und die Futterausnutzung bei Tieren und sind als Antioxidantien verwendbar.

Die erfindungsgemässen Verbindungen weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemässen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemässen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes und Gaffkya tetragena (Staph. = Staphylococcus);

Lactobacteriaceae, wie Streptokokken, z.B. Streptococcus pyogenes, α- bzw. β-hämolysierende Streptokokken, nicht (γ-)-hämolysierende Streptokokken, Str. viridans, Str. faecalis (Enterokokken) und Diplococcus pneumoniae (Pneumokokken) (Str. = Streptococcus);

Enterobacteriaceae, wie Escherichiae-Bakterien der Coli-Gruppe: Escherichia-Bakterien, z.B. Escherichia coli, Enterobacter-Bakterien, z.B. E. aerogenes, E. cloacae, Klebsiella-Bakterien, z.B. K. pneumoniae, Serratia, z.B. Serratia marcescens (E. = Enterobacter) (K. = Klebsiella), Proteae-Bakterien der Proteus-Gruppe: Proteus, z.B. Proteus vulgaris, Pr. morganii, Pr. rettgeri, Pr. mirabilis, (Pr. = Proteus);

Pseudomonadaceae, wie Pseudomonas-Bakterien, z.B. Pseudomonas aeruginosa, (PS. = Pseudomonas);

Bacteroidaceae, wie Bacteroides-Bakterien, z.B. Bacteroides fragilis, (B. = Bacteroides).

Die obige Aufzählung von Erregern ist lediglich beispielhaft und keineswegs beschränkend aufzufassen.

Als Krankheiten, die durch die erfindungsgemässen Verbindungen verhindert, gebessert und/oder geheilt werden können seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemässe Verbindungen enthalten oder die aus einem oder mehreren erfindungsgemässen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, sie (a) Füll- und Streckmittel, z.B. Stärken, Milchzucker, Rohrzukker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können ausser den erfindungsgemässen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 5 bis etwa 1000, vorzugsweise 10 bis 200 mg/kg Körpergewicht je 24 Stunden, gegebenenfalls in Form mehrerer Einzelgaben, zur Erzielung der gewünschten Ergebnsse zu verabreichen. Eine Einzelgabe enthält den oder die erfindungsgemässen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen Verbindungen zeichnen sich durch starke antibakterielle Wirkungen, die in vivo und in vitro geprüft wurden, und durch orale Resorbierbarkeit aus.

Die erfindungsgemässen Verbindungen können zum Zwecke der Erweiterung des Wirkungsspektrums und um eine Wirkungssteigerung speziell bei β-lactamasebildenden Bakterien zu erzielen mit anderen antimikrobiellen Wirkstoffen z.B. mit Penicillinen, die besonders penicillinasefest sind, kombiniert werden. Eine solche Kombination wäre z.B. die mit Oxacillin oder Dicloxacillin.

Die erfindungsgemässen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

Die erfindungsgemässen Verbindungen verhindern den Abbau von z.B. Penicillinen durch β-Lactamasen, die normalerweise empfindliche Penicilline hydrolysieren und damit unwirksam machen. Gibt man zu einem rohen Extrakt von β-Lactamase aus E. coli A 261 eine Menge von 100 mg Mezlocillin pro Milliliter, dann wird das Penicillin in 60 min. völlig inaktiviert. Gibt man aber eine Kombination von 100 mg Mezlocillin plus 100 mg der Verbindung aus Beispiel 23, dann bleibt Mezlocillin voll antibakteriell wirksam.

Die Wirksamkeit der erfindungsgemässen β-Lactamantibiotica kann durch die folgenden in vitro-Versuche beispielhaft demonstriert werden:

In vitro-Versuche

Die Verbindungen der Beispiele 18 und 23, welche als typische Vertreter der erfindungsgemässen Verbindungen betrachtet werden können, wurden im Agarverdünnungstest unter deutschen DIN-Norm-Bedingungen auf antibakterielle Wirkung geprüft. Die Konzentration betrug 100 mcg pro Milliliter Agar. Bei folgenden Bakterienstämmen wurde völlige Wachstumshemmung festgestellt:

E. coli T 7, Klebsiella 57 US, Serratia marcescens 16001, Providencia 12012, Proteus morganii 932, Proteus vulgaris 1017, Proteus rettgeri, Enterobacter aerogenes 55 US, Pseudomonas aeruginosa W, Bacteroides fragilis 012 999, Staphylococcus aureus 133, Enterococcus ATCC 9790.

Beispiele

Die Bestimmung der Doppelbindungskonfiguration in den erfindungsgemässen Verbindungen erfolgte mit Hilfe der $^{13}C$-Kernresonanzspektroskopie. Es ist bekannt, dass bei trisubstituierten Olefinen des Typs XII

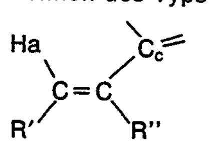

XIIa

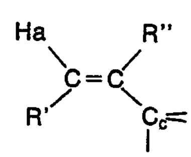

XIIb

worin R′ und R″ organische Reste darstellen, die $^{3}J$-Ha $C_c$-Heterokopplung bei trans-Stellung von Ha und $C_c$ (XIIb) immer wesentlich grösser ist als bei der entsprechenden cis-Stellung (XIIa). Diese Beobachtung wurde zur Konfigurationszuordnung an einer der Vorstufen V, VIII oder II der erfindungsgemässen Verbindungen verwendet.

Beispiel 1

2-(2-Bromo-2-phenyl)-methyl-3-oxo-4-bromobuttersäureethylester

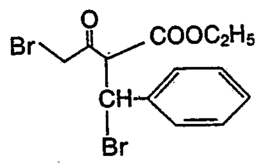

Zu 595 g 2-Benzylidenacetessigsäureethylester in 3 l Methylenchlorid werden bei −10 bis −5° 430 g Brom getropft. Man rührt eine Stunde bei Raumtemperatur nach, zieht dann das Lösungsmittel bei Raumtemperatur ab, versetzt den Rückstand mit Ligroin und dekantiert. Der Rückstand wird erneut mit Ligroin versetzt und die Mischung über Nacht stehen gelassen. Das ausgefallene Produkt wird abgesaugt und zweimal aus Ligroin umkristallisiert. Ausbeute: 23,5%, Fp.: 99–101°.

IR (Nujol): 1738, 1712, 1575, 1460, 1320, 1290, 1270, 1208, 1181, 1143, 1092, 1030, 1005 $cm^{-1}$.

NMR ($CDCl_3$): δ = 0,91[3] t, J = 7 Hz, 3,92[2] q, J = 7 Hz, 4,25[2] s, 4,83[1] d, J = 11 Hz, 5,54[1] d, J = 11 Hz, 7,36[5] m ppm.

Beispiel 2

2-(2-Aminothiazol-4-yl)-3-phenylpropensäureethylester. (E-Isomeres).

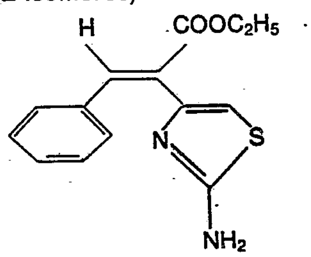

323,5 g der nach Beispiel 1 hergestellten Substanz werden in 5 l Ethanol gelöst. Zu dieser Lösung werden bei 50° 65 g Thioharnstoff portionsweise eingetragen. Man rührt 2 h bei 50° nach, zieht das Lösungsmittel ab und nimmt den öligen Rückstand in Wasser auf. Nach Einstellen auf pH 9 mit konz. Ammoniaklösung wird dreimal mit Essigester extrahiert. Man trocknet die gereinigten Extrakte über Magnesiumsulfat und dampft ein. Der Rückstand wird aus Ethanol und Acrton umgelöst. Ausbeute: 45%, Fp.: 162°.

IR (Nujol): 1680, 1620, 1520, 1365, 1260, 1205 $cm^{-1}$.

NMR ($CDCl_3$ + DMSO): δ = 7,75[1] s, 7,22[5] s, 6,25[1] s, 6,19[2] s breit, 4,21[2] q, J = 7 Hz, 1,25[3] t, J = 7 Hz ppm.

| | | | | |
|---|---|---|---|---|
| ber.: | C 61,3 | H 5,1 | N 10,2 | S 11,7 |
| gef.: | C 61,0 | H 5,3 | N 10,2 | S 11,2 |

Beispiel 3

2-(2-Aminothiazol-4-yl)-3-phenylpropensäure (E-Isomeres)

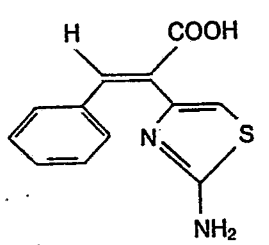

20 g der nach Beispiel 2 hergestellten Verbindung, gelöst in 250 ml Methanol, werden zu 222 ml 1 n Natronlauge gegeben. Nach Rühren bei Raumtemperatur über Nacht wird das Methanol abgezogen. Die zurückbleibende wässrige Phase stellt man mit 2 n Salzsäure auf pH 6,5 ein, extrahiert zweimal mit Essigester und stellt dann langsam auf pH 4 ein. Das ausgefallene Produkt wird abgesaugt und aus Ethanol umgelöst. Ausbeute: 75,2%, Fp.: 206–7° (Zers.).

IR (Nujol): 1660, 1620, 1602, 1589, 1321, 1226, 1196 $cm^{-1}$.

NMR ($CDCl_3$ + DMSO): δ = 7,67[1] s, 7,23[5] s, 6,90[2] s breit, 6,25[1] s ppm.

| | | | | |
|---|---|---|---|---|
| ber.: | C 58,5 | H 4,1 | N 11,4 | S 13,0 |
| gef.: | C 58,0 | H 4,2 | N 11,2 | S 12,7 |

Beispiel 4

2-(2,6-Dichlorbenzyliden)-3-oxo-4-chlorbuttersäureethylester

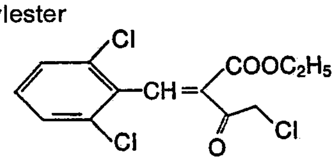

10 g 4-Chloracetessigsäureethylester, 10,5 g 2,6-Dichlorbenzaldehyd, 0,7 ml Eisessig und 0,35 ml Piperidin werden in 15 ml Benzol gelöst. Man kocht die Lösung 6 Stunden am Wasserabscheider, lässt dan abkühlen, versetzt mit 100 ml Ether und extrahiert je einmal mit gesättigter Natriumbicarbonatlösung, Wasser, 1 n Zitronensäurelösung und erneut mit Wasser. Die organische Phase wird über Magnesiumsulfat getrocknet und eingeengt. Das zurückbleibende Öl wird im Hochvakuum andestilliert. Als Rückstand verbleibt das Produkt als Öl, welches die beiden Isomeren nach GC und NMR als 3:1-Gemisch enthält. Ausbeute: 64,5%.

Aus diesem Gemisch lässt sich mit Isopropanol das als Hauptkomponente vorliegende Z-Isomere rein kristallisieren. 5,5 g, Fp.: 74–6°. In der Mutterlauge verbleibt ein nicht mehr kristallisierbares 1:1-E/Z-Gemisch.

a) Z-Isomeres:

IR (KBr): 1727, 1708, 1619, 1557, 1441, 1430, 1377, 1232 $cm^{-1}$.

NMR ($CDCl_3$): δ = 7,82[1] s, 7,32[3] m, 4,60[2] s, 4,12[2] q, J = 7 Hz, 0,98[3] t, J = 7 Hz ppm.

| | | | |
|---|---|---|---|
| ber.: | C 48,6 | H 3,4 | Cl 33,1 |
| gef.: | C 48,4 | H 3,4 | Cl 33,1 |

b) E-Isomeres:

NMR ($CDCl_3$): δ = 7,80[1] s, 7,32[3] m, 4,43[2] s, 4,40[2] g, J = 7 Hz, 1,38[3] t, J = 7 Hz ppm.

Beispiel 5

2-(2-Aminothiazol-4-yl)-3-(2,6-dichlorphenyl)-propensäureethylesterhydrochlorid (Z-Isomeres)

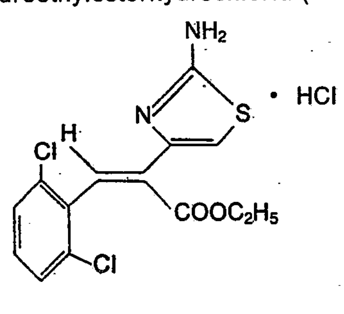

52,2 g des nach Beispiel 4 hergestellten rohen E/Z-Gemischs und 12,3 g Thioharnstoff werden in 500 ml Ethanol gelöst. Die Lösung wird nach 5 Stunden auf 50 °C erhitzt und nach 16 Stunden bei Raumtemperatur stehengelassen. Nach Absaugen vom Produkt lässt sich durch Einengen der Mutterlauge eine zweite Kristallfraktion gleicher Reinheit kristallisieren. Das Produkt wird aus Ethanol umgelöst. Ausbeute: 36,5%, Fp: 250 °C.

IR (Nujol): 1710, 1635, 1588, 1315, 1240, 1160, 1020 $cm^{-1}$.

NMR (DMSO): δ = 8,41[3] s breit, 7,62[1] s, 7,50[3] m, 7,07[1] s, 4,04[2] q, J = 7 Mz, 0,91[3] t, J = 7 Hz ppm.

| | | | | | |
|---|---|---|---|---|---|
| ber.: | C 44,3 | H 3,4 | N 7,4 | S 8,4 | Cl 28,1 |
| gef.: | C 44,2 | H 3,6 | N 6,4 | S 8,0 | Cl 27,8 |

Beispiel 6

2-(2-Aminothiazol-4-yl)-3-(2,6-dichlorphenyl)-propensäure (Z-Isomeres)

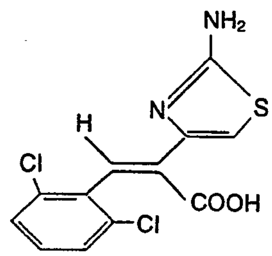

Eine Lösung von 10 g des nach Beispiel 5 hergestellten Esters in einem Gemisch aus 50 ml Dioxan und 20 ml Wasser wird mit 6,5 g 45prozentiger Natronlauge versetzt und dann 56 Stunden am Rückfluss getrocknet. Man versetzt mit 100 ml Wasser, zieht das Dioxan ab und stellt die wässrige Phase mit 2 n Salzsäure auf pH 7,5 ein. Nach zweimaliger Extraktion mit Essigester wird auf pH 2 eingestellt und das ausgefallene Produkt abgesaugt. Ausbeute: 88,7%, Fp.: 163–65° (Zers.).

IR (Nujol): 1642, 1601 (Schulter), 1580, 1405, 1285, 1198 $cm^{-1}$.

NMR (DMSO): δ = 7,45[4] m, 7,16[2] s, breit, 6,90[1] s ppm.

Beispiel 7

2-(4-Chlorbenzyliden)-3-oxo-4-chlorbuttersäure-ethylester

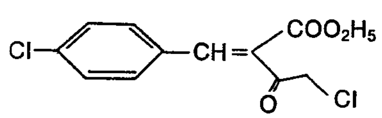

25,7 g 4-Chloracetessigsäureethylenester, 23,9 g 4-Chlorbenzaldehyd, 0,7 ml Piperidin und 18 ml Eisessig werden in 15 ml Benzol gelöst. Die Lösung kocht man 5 Stunden am Wasserabscheider, lässt abkühlen und versetzt mit 300 ml Ether. Nach dreimaligem Waschen mit Wasser wird über Magnesiumsulfat getrocknet und das Lösungsmittel abgedampft. Der Rückstand wird im Hochvakuum destilliert. Das anfallende Produkt ist ein Z/E-Gemisch im Verhältnis 3/1. Ausbeute: 23,4%, $Kp_{0,5}$: 161–65°.

IR (Film): 1720, 1695 (Schulter), 1622, 1592, 1494, 1395, 1315, 1290, 1260, 2000 $cm^{-1}$.

NMR ($CDCl_3$):

a) Z-Isomeres α = 7,75[1] s, 7,30[4] s, 4,32[2] s, 4,30[2] q, J = 7 Hz, 1,31[3] t, J = 7 Hz ppm.

b) E-Isomeres α = 7,66[1] s, 7,36[4] s, 4,50[2] s, 4,30[2] q, J = 7 Hz, 1,25[3] t, J = 7 Hz ppm.

| | | | |
|---|---|---|---|
| ber.: | C 54,4 | H 4,2 | Cl 24,7 |
| gef.: | C 54,0 | H 4,2 | Cl 24,8 |

Beispiel 8

2-(2-Aminothiazol-4-yl)-3-(4-chlorphenyl)-propensäureethylester

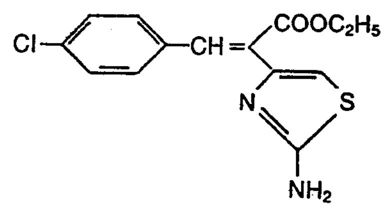

10,5 g der nach Beispiel 7 hergestellten Substanz und 2,8 g Thioharnstoff werden in 275 ml Ethanol gelöst und 2 Stunden auf 50° erwärmt. Nach Abdampfen des Lösungsmittels wird das zurückbleibende Öl mit Essigester und Wasser versetzt und die Lösung mit Salzsäure auf pH 1 eingestellt. Man trennt den Essigester ab, trocknet ihn über Magnesiumsulfat und dampft das Lösungsmittel ab. Das zurückbleibende zähe Öl besteht aus verunreinigtem Produkt. Die saure Wasserphase wird mit konz. Ammoniaklösung auf pH 11 eingestellt und dann dreimal mit Chloroform extrahiert. Nach Trocknen über Magnesiumsulfat und Eindampfen des Chloroforms lässt sich das reine E-Isomere des Produkts mit Ethanol kristallisieren. Durch Mitteldruck-Chromatographie der Mutterlauge kann weiteres E-Isomeres und auch Z-Isomeres der Titelverbindung in jeweils reiner Form isoliert werden.

a) E-Isomeres:

Fp.: 119–21° (Zers.)

IR (Nujol): 3350, 3240, 3080, 1695, 1630, 1620, 1530, 1490, 1270, 1205, 1182, 1098 $cm^{-1}$.

NMR ($CDCl_3$): α = 7,88[1] s, 7,26[4] m, 6,42[4] s, 5,50[2] s breit, 4,33[2] q, J = 7 Hz, 1,33[3] t, J = 7 Hz ppm.

| | | | | | |
|---|---|---|---|---|---|
| ber.: | C 54,5 | H 4,2 | N 9,1 | S 10,4 | Cl 11,5 |
| gef.: | C 53,4 | H 4,5 | N 9,2 | S 10,8 | Cl 11,4 |

b) Z-Isomeres:

Fp.: 114–16° (Zers.)

IR (Nujol): 3360, 3250, 3100, 1698, 1630, 1535, 1222, 1100, 1030, 1010 $cm^{-1}$.

NMR ($CDCl_3$): δ = 7,45[1] s, 7,26[4] m, 6,55[1] s, 5,20[2] s breit, 4,25[2] q, J = 7 Hz, 1,18[3] t, J = 7 Hz ppm.

| | | | | | |
|---|---|---|---|---|---|
| ber.: | C 54,5 | H 4,2 | N 9,1 | S 10,4 | Cl 11,5 |
| gef.: | C 53,5 | H 4,3 | N 8,6 | S 9,8 | Cl 11,8 |

Beispiel 9
2-(2-Aminothiazol-4-yl)-3-(4-chlorphenyl)-propensäure (E-Isomeres)

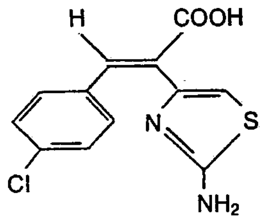

10 g des nach Beispiel 8 hergestellten reinen E-Isomeren werden in einem Gemisch aus 50 ml Methanol und 20 ml Wasser gelöst. Die Lösung wird mit 20 ml 2 n Natronlauge versetzt und über Nacht bei Raumtemperatur gerührt. Man zieht das Methanol ab, stellt die wässrige Phase mit 2 n Salzsäure auf pH 3 ein und saugt das ausgefallene Produkt ab. Es wird aus Ethanol umkristallisiert. Ausbeuge: 66,6%, Fp.: 225° (Zers.).

IR (Nujol): 1650, 1620, 1585, 1338 $cm^{-1}$.

NMR (DMSO): α = 7,72[1] s, 7,33[4] m, 7,0–7,8[3] breit, 6,40[1] s ppm.

Beispiel 10
2-(2-Furyliden)-3-oxo-4-chlorbuttersäureethylester

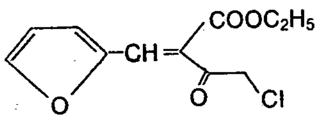

200 g 4-Chloracetessigsäureethylester, 125,6 g frisch destilliertes Fururol, 2,6 ml Piperidin und 7 ml Eisessig werden in 120 ml Benzol gelöst, 5 Stunden am Wasserabscheider gekocht. Die abgekühlte Lösung wird mit 1 l Ether versetzt und dann je einmal mit gesättigter Natriumbicarbonatlösung, Wasser, 2 m Zitronensäurelösung und wieder mit Wasser ausgeschüttelt. Nach Trocknen über Magnesiumsulfat und Einengen erhält man ein Öl, das im Hochvakuum andestilliert wird. Das Produkt bleibt als zähes Öl zurück, das E/Z-Isomeren-Verhältnis beträgt 4:1. Ausbeute: 86,8%.

IR (Nujol): 1720, 1678, 1620, 1450, 1415, 1355, 1303 $cm^{-1}$.

NMR ($CDCl_3$): Signale äquivalenter Protonen aus isomeren Verbindungen sind durch einen / getrennt δ = 7,50[2] m, 7,03/6,89[1] d, J = 7 Hz, 6,57[1] m, 4,48/4,50[2] s, 4,43/4,32[2] q, J = 7 Hz, 1,40/1,34[3] t, J = 7 Hz ppm.

| | | | |
|---|---|---|---|
| ber.: | C 54,4 | H 4,6 | Cl 14,6 |
| gef.: | C 54,3 | H 4,5 | Cl 14,8 |

Beispiel 11
2-(2-Aminothiazol-4-yl)-3-(2-furyl)-propensäureethylesterhydrochlorid (E-Isomeres)

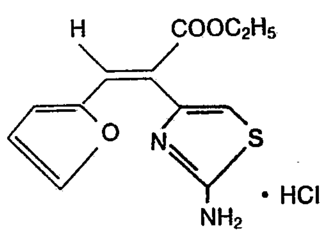

32,2 g des nach Beispiel 10 hergestellten Isomerengemischs und 10 g Thioharnstoff werden in 850 ml Ethanol gelöst. Die Lösung erhitzt man 4 Stunden auf 50° und lässt dann 16 Stunden bei Raumtemperatur stehen. Das ausgefallene Produkt wird abgsaugt. Durch Einengen der Mutterlauge lässt sich weiteres Produkt gleicher Reinheit gewinnen. Nach Umkristallisation aus Ethanol erhält man das E-Isomere, das etwa 10% des Z-Isomeren enthält. Ausbeute: 65,4%, Fp.: > 250°. Durch wiederholte Kristallisation aus Ethanol lässt sich das E-Isomere rein gewinnen.

IR (Nujol): 1679, 1620, 1300, 1270, 1222, 1156, 1080, 1050, 1025 $cm^{-1}$.

NMR (DMSO): δ = 6,6–8,2[2] breit, NH, 7,86[1] d, J = 2 Hz, 7,75[1] s, 7,0[1] d, J = 4 Hz, 6,90[1] s, 6,66[1] dd, J = 2 Hz, J = 4 Hz, 4,20[2] q, J = 7 Hz, 1,25[3] t, J = 7 Hz ppm.

| | | | | | |
|---|---|---|---|---|---|
| ber.: | C 47,9 | H 4,4 | N 9,3 | S 10,7 | Cl 11,8 |
| gef.: | C 47,8 | H 4,5 | N 9,1 | S 10,2 | Cl 11,7 |

Beispiel 12
2-(2-Aminothiazol-4-yl)-3-(2-furyl)-propensäure (E-Isomeres)

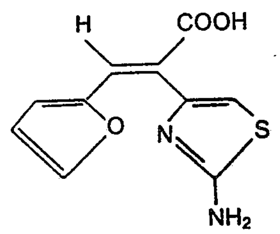

8,5 g des nach Beispiel 11 hergestellten Produkts werden in einem Gemisch aus 50 ml Methanol und 50 ml Wasser gelöst. Die Lösung bringt man mit 2 n Natronlauge auf pH 13 und rührt 5 Stunden bei Raumtemperatur. Anschliessend wird das Methanol abgezogen, die wässrige Lösung mit 2 n Salzsäure auf pH 7,5 gestellt und zweimal mit Essigester extrahiert. Man stellt auf pH 3, saugt das Produkt ab und kristallisiert aus Ethanol um. Ausbeute: 64,3%, Fp.: 201° (Zers.).

IR (Nujol): 1630, 1598, 1570, 1540, 1330, 1280 $cm^{-1}$.

NMR (DMSO): δ = 7,84[1] d, J = 1,5 Hz, 7,64[1] s, 7,50[3] s breit, 6,65[1] s, 6,61[2] s, verbreitert ppm.

| | | | | |
|---|---|---|---|---|
| ber.: | C 50,8 | H 3,4 | N 11,9 | S 13,5 |
| gef.: | C 50,4 | H 3,4 | N 11,6 | S 12,8 |

Beispiel 13
2-(3-Thienyliden)-3-oxo-4-chlorbuttersäureethylester

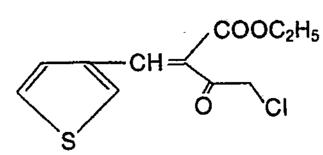

55,3 g 4-Chloracetessigsäureethylester, 41 g Thiophen-3-aldehyd, 3,8 ml Eisessig und 1,5 ml Piperidin werden in 30 ml Benzol gelöst. Man kocht die Lösung 7 Stunden am Wasserabscheider, gibt nach dem Abkühlen 400 ml Ether zu und extrahiert dreimal mit Wasser. Nach Trocknen über Natriumsulfat und Abdampfen des Lösungsmittels wird das Rohprodukt andestilliert und der Rückstand anschliessend über eine kurze Kieselgelsäure (70–230mal) gereinigt. (Laufmittel Petrolether/Ether 8/2). Man erhält E/Z-Gemisch als Öl. Ausbeute: 63%.

IR (Film): 1720, 1690, 1609, 1265, 1240 $cm^{-1}$.

NMR ($CDCl_3$): δ = 7,54–7,83[2] m, 6,95–7,50[2] m, 4,52/4,45[2] s, 4,35/4,26[2] q, J = 7 Hz, 1,27/1,32[3] t, J = 7 Hz ppm.

Die einander entsprechenden Signale isomerer Verbindungen sind durch einen / getrennt.

| | | | | |
|---|---|---|---|---|
| ber.: | C 51,1 | H 4,3 | S 12,4 | Cl 13,7 |
| gef.: | C 51,7 | H 4,5 | S 12,4 | Cl 12,6 |

Beispiel 14

2-(2-Aminothiazol-4-yl)-3-(3-thienyl)-propensäureethylesterhydrochlorid (E-Isomeres)

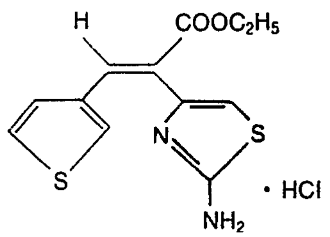

22 g des in Beispiel 13 hergestellten E/Z-Gemischs und 6,5 g Thioharnstoff werden, gelöst in 100 ml Ethanol, 2,5 Stunden auf 50° erhitzt. Die Lösung wird eingedampft, der Rückstand mit wenig Wasser und Essigester versetzt und das Produkt abgesaugt. Ausbeute: 22%, Fp.: 213–15° (Zers.).

IR (Nujol): 1685, 1620, 1278 $cm^{-1}$.

NMR ($CD_3OD$): δ = 8,18[1] s, 7,87[1] dd, J = 2 Hz, J = 4 Hz, 7,53[1] dd, J = 4 Hz, J = 6 Hz, 6,95[1] dd, J = 2 Hz, J = 6 Hz, 6,86[1] s, 4,32[2] q, J = 7 Hz, 1,32[3] t, J = 7 Hz ppm.

| | | | | | |
|---|---|---|---|---|---|
| ber.: | C 45,5 | H 4,1 | N 8,8 | S 20,2 | Cl 11,2 |
| gef.: | C 45,1 | H 4,4 | N 8,5 | S 19,5 | Cl 10,9 |

Beispiel 15

2-(2-Aminothiazol-4-yl)-3-(3-thienyl)-propensäure (E-Isomeres)

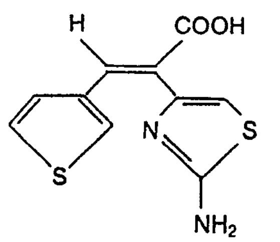

3,3 g des nach Beispiel 14 hergestellten Produkts, gelöst in 10 ml Methanol, werden mit 8 ml 2 n Natronlauge versetzt. Man lässt die Lösung über Nacht bei Raumtemperatur stehen, zieht dann das Methanol ab und löst den Rückstand in Wasser. Mit 2 n Salzsäure wird pH 8 eingestellt und die wässrige Lösung dreimal mit Essigsäure extrahiert. Anschliessend fällt man das Produkt durch Einstellen auf pH 3 aus und saugt ab. Ausbeute: 50,5%, Fp.: 172–75° (Zers.).

IR (KBr): 1628, 1367, 1262, 1028 $cm^{-1}$.

NMR (DMF): α = 8,5, N–H, sehr breit, 7,93[1] s, verbreitet, 7,86[1] dd, $J_1$ = 1,5 Hz, $J_2$ = 3 Hz, 7,54[1] ddd, $J_3$ = 1 Hz, $J_4$ = 6 Hz, 6,91[1] dd, $J_4$ = 6 Hz, $J_1$ = 1,5 Hz, 6,70[1] s ppm.

| | | | | |
|---|---|---|---|---|
| ber.: | C 47,6 | H 3,2 | N 11,1 | S 25,4 |
| gef.: | C 47,2 | H 3,1 | N 11,4 | S 25,3 |

Beispiel 16

E-Natrium-7-[2-(2-aminothiazol-4-yl)-2-benzylidenacetamido]-3-acetoximethyl-3-cefem-4-carboxylat

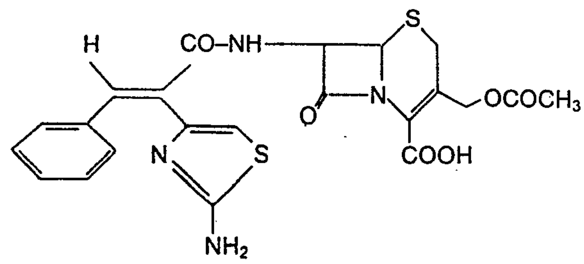

Eine Suspension von 4,9 g des nach Beispiel 3 hergestellten Produkts in 200 ml Essigester wird auf 0–5° abgekühlt und mit 3,8 g Phosphoroxichlorid versetzt. Nach 30 Minuten setzt man 3,3 g Trimethylsilylacetamid, gelöst in 5 ml Essigester, zu und anschliessend erneut 3,8 g Phosphoroxichlorid. Es wird 15 Minuten bei 0° nachgerührt und dann mit 1,8 ml Dimethylformamid versetzt. Nach weiteren 40 Minuten wird auf −10° abgekühlt und zu einer Lösung von 5,44 g 7-Aminocefalosporansäure und 4 g Natriumhydrogencarbonat in einem Gemisch aus 100 ml Wasser und 75 ml Aceton, die auf −5° gekühlt wurde, getropft. Man rührt 2 Stunden nach, wobei der pH-Wert mit gesättigter Natriumhydrogencarbonatlösung auf pH 6 gehalten wird. Die Lösung wird filtriert, die wässrige Phase abgetrennt und einmal mit Essigester gewaschen, mit Essigester versetzt, und auf pH 3,5 eingestellt. Der Essigester wird abgetrennt und die wässrige Phase noch zweimal mit Essigester extrahiert. Die vereinigten Essigesterphasen werden mit Wasser versetzt und auf pH 7 gebracht. Man trennt die wässrige Phase ab und lyophilisiert, es werden 2,0 g des Produkts erhalten.

IR (Nujol): 1750 $cm^{-1}$.

NMR ($CD_3OD$). δ = 7,78[1] s, 7,29[5] s, 6,32[1] s, 5,82[1] d, J = 5 Hz, 5,09[1] d, J = 5 Hz, 5,06[1] d, J = 14 Hz, 4,83[1] d, J = 14 Hz, 3,60[1] d, J = 18 Hz, 3,29[1] d, J = 18 Hz, 2,03[3] s ppm.

Beispiel 17

E-Natrium-6-[2-(2-aminothiazol-4-yl)-2-benzylidenacetamido]-penam-3-carboxylat

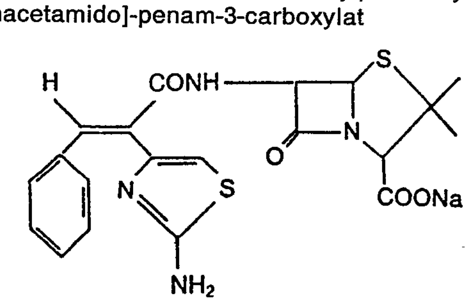

1,9 g des Produkts aus Beispiel 3 werden entsprechend der Vorschrift aus Beispiel 16 mit 6-Aminopenicillansäure anstelle von 7-Aminocefalosporansäure umgesetzt. Man erhält 1,4 g lyophilisiertes Natriumsalz. Fp.: 250°.

IR (KBr): 1761, 1607, 1508 $cm^{-1}$.

NMR ($CD_3OD$): 7,78[1] s, 7,25[5] s, 6,33[1] s, 5,62[2] s, 4,25[1] s, 1,58[6] s ppm.

Beispiel 18

Z-Trimethylammonium-7-[2-aminothiazol-4-yl)-2-(2,6-dichlorbenzyliden)-acetamido]-3-acetoximethyl-3-cefem-4-carboxylat

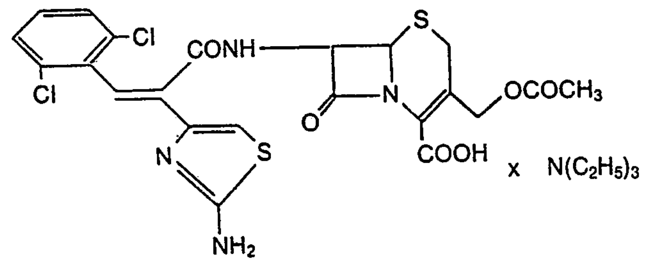

Eine Lösung von 2,1 g des Produkts aus Beispiel 6 in 15 ml abs. Dimethylformamid wird mit 0,68 g 1-Hydroxibentriazol und 1,03 g N,N'-Dicyclohexylcarbodiimid versetzt und 5 Stunden bei Raumtemperatur gerührt. Man saugt ab und tropft zur Mutterlauge eine Lösung von 1,36 g 7-Aminocefalosporansäure und 2,0 ml Triethylamin in 25 ml Methylenchlorid. Die Reaktionslösung wird über Nacht bei Raumtemperatur gerührt und dann zur Trockne eingedampft. Der Rückstand wird in wenig Wasser gelöst und die Lösung bei pH 7 abgesaugt und anschliessend mehrfach mit Essigester gewaschen. Man stellt die wässrige Lösung mit 2 n Salzsäure auf pH 4,5 ein, saugt das ausgefallene Produkt ab, wäscht mit Wasser und trocknet über Kaliumhydroxid. Das Rohprodukt wird 1 Stunde mit Essigester verrührt, abgesaugt, der Rückstand 1 Stunde mit Methanol verrührt, erneut abgesaugt und das Filtrat kalt zur Trockne eingedampft. Der Rückstand wird über Kaliumhydroxid getrocknet. Man erhält 0,6 g des gewünschten Produkts.

IR (KBr): 1775, 1735, 1675, 1620 $cm^{-1}$.

NMR ($CD_3OD$): $\delta$ = 7,2–7,44[4] m, 6,78[1] s, 5,71[1] d, J = 6 Hz, 5,07[1] d, J = 12 Hz, 5,05[1] d, J = 6 Hz, 4,82[1] d, J = 12 Hz, 3,60[1] d, J = 18 Hz, 3,38[1] d, J = 18 Hz, 2,07[3] s; 3,23[6] q, J = 7 Hz und 1,32[9] t, J = 7 Hz, ppm.

Beispiel 19

E-7[2-(2-Aminothiazol-4-yl)-2-(2-furyliden)acetamido]-3-acetoximethyl-3-cefem-4-carbonsäure

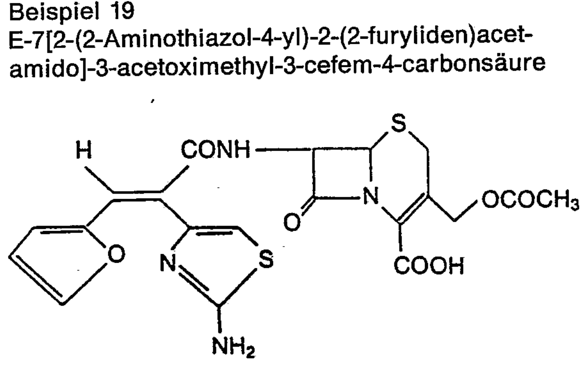

2,0 g des nach Beispiel 12 hergestellten Produkts werden über Stickstoff im vorher ausgeheizten Kolben in 30 ml abs. Dimethylformamid gelöst. Man versetzt die Lösung mit 1,12 g 1-Hydroxibenztriazol und 1,7 g N,N'-Dicyclohexylcarbodiimid und rührt 6 h bei Raumtemperatur. Nach Absaugen von Dicyclohexylharnstoff wird zum Filtrat eine Lösung von 2,2 g 7-Aminocefalosporansäure und 3,4 ml Triethylamin in 20 ml Methylenchlorid getropft. Es wird über Nacht gerührt, dann zur Trockne eingedampft und der Rückstand bei pH 7 gelöst. Nach mehrmaligem Extrahieren mit Essigester wird die wässrige Lösung mit 2 n HCl auf pH 2,5 gestellt und 30 Min. gerührt. Das Produkt wird abgesaugt und über KOH getrocknet. Dieses Rohprodukt wird 1 Stunde mit Essigester verrührt, abgesaugt und dann in kaltem Methanol gelöst. Durch Filtrieren und Eindampfen der Lösung wird das Produkt gewonnen.

IR (KBr): 1759, 1612, 1514, 1397, 1249 $cm^{-1}$.

NMR ($CD_3OD$): $\delta$ = 7,54[1] s, 7,52[1] s, 6,60[1] s, 6,44[2] m, 5,76[1] d, J = 5 Hz, 5,04[1] d, J = 5 Hz, 4,98[1] d, J = 11 Hz, 4,80[1] d, J = 11 Hz, 3,58[1] d, J = 17 Hz, 3,30[1] d, J = 17 Hz, 2,01[3] ppm.

Beispiel 20

E-Natrium-7-[2-(2-aminothiazol-4-yl)-2-(3-thienyliden)acetamido]-3-acetoximethyl-3-cefem-4-carboxylat

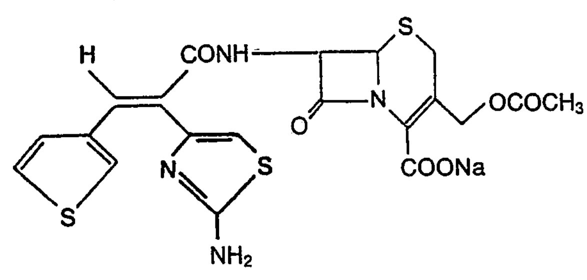

1,0 g des nach Beispiel 15 hergestellten Produkts wird gemäss Beispiel 18 umgesetzt. Die bei pH 4,5 gefällte freie Säure wird bei pH 6,5 mit 2 n Natronlauge in Wasser in Lösung gebracht, anschliessend wird die Lösung lyophilisiert. Man erhält 0,7 g der gewünschten Verbindung.

IR (Nujol): 1758, 1605, 1412, 1220 $cm^{-1}$.

NMR ($CD_3OD$): $\delta$ = 7,76[1] s, 7,52[1] d, J = 2 Hz, 7,29[1] dd, J = 2 Hz, J = 6 Hz, 6,79[1] d, J = 6 Hz, 5,79[1] d, J = 5 Hz, 5,08[1] d, J = 5 Hz, 5,00[1] d,

J = 11 Hz, 4,82[1] d, J = 11 Hz, 3,60[1] d, J = 18 Hz, 3,32[1] d, J = 18 Hz, 2,04[3] s ppm.

Beispiel 21
E-Natrium-7-[2-(2-aminothiazol-4-yl)-2-(4-chlorbenzyliden)-acetamido]-3-acetoximethyl-3-cefem-4-carboxylat

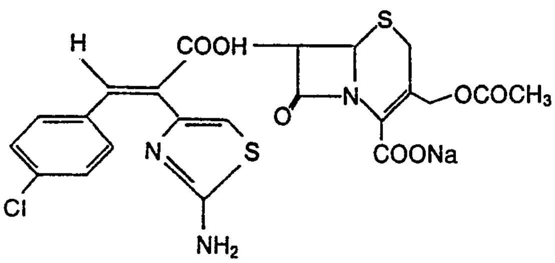

2,0 g der nach Beispiel 9 hergestellten Verbindung werden gemäss Beispiel 19 umgesetzt. Das bei pH 2,5 abgesaugte Rohprodukt wird in kaltem Methanol gelöst, die Lösung wird filtriert und zur Trockne eingedampft. Der Rückstand wird in Wasser bei pH 6,8 mit 2 n Natronlauge in Lösung gebracht. Durch Lyophilisieren der wässrigen Lösung gewinnt man 0,9 g des gewünschten Produkts.

IR (Nujol): 1759, 1610, 1515 $cm^{-1}$.

NMR ($CD_3OD$): δ = 7,67[1] s, 7,25[4] m, 6,35[1] s, 5,82[1] d, J = 5 Hz, 5,10[1] d, J = 5 Hz, 5,02[1] d, J = 12 Hz, 4,85[1] d, J = 12 Hz, 3,61[1] d, J = 17 Hz, 3,33[1] d, J = 17 Hz, 2,06[3] s ppm.

Beispiel 22
E-Natrium-7-[2-(2-aminothiazol-4-yl)-2-(4-chlorbenzyliden)-acetamido]-penam-3-carboxylat

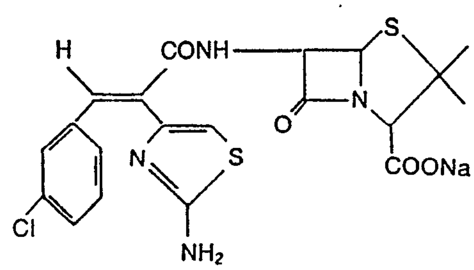

2,3 g des Produkts aus Beispiel 9 werden gemäss Beispiel 17 umgesetzt. Man erhält 1,0 g lyophilisiertes Natriumsalz.

IR (Nujol): 1755, 1600 $cm^{-1}$.

NMR ($CD_3OD$): δ = 7,71[1] s, 7,24[4] m, 6,36[1] s, 5,59[2] s, 4,20[1] s, 1,55[3] s, 1,54[3] s ppm.

Beispiel 23
Z-6-[2-(2-Aminothiazol-4-yl)-2-(2,6-dichlorbenzyliden)-acetamido]-penam-3-carbonsäure

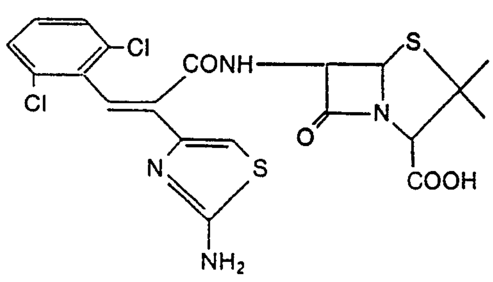

0,9 g des Produkts aus Beispiel 6 werden in 15 ml abs. DMF gelöst. Man gibt 0,3 g Hydroxibenztriazol und 0,44 g DCC zu und rührt 5 Stunden bei Raumtemperatur. Es wird abgesaugt und die Mutterlauge zu einer Lösung von 0,4 g 6-Aminopenicillansäure und 0,9 ml Triethylamin in 25 ml Methylenchlorid getropft. Nach Rühren bei Raumtemperatur über Nacht dampft man ein, versetzt den Rückstand mit Wasser und Essigester und stellt auf pH 6,5 ein. Es wird abgesaugt, die organische Phase abgetrennt und die wässrige Phase noch einmal mit Essigester gewaschen. Anschliessend stellt man auf pH 2,8 ein und saugt ab. Es werden so 0,5 g Produkt gewonnen.

IR (KBr): 1770 (Schulter), 1728, 1637 $cm^{-1}$.

NMR ($CD_3OD$): δ = 7,1[4] m, 6,74[1] s, 5,52[1] d, J = 6 Hz, 5,45[1] d, J = 6 Hz, 4,31[1] s, 1,49[3] s, 1,47[3] s ppm.

Beispiel 24
2-(2,4,6-Trimethylbenzyliden)-3-oxo-4-chlorbuttersäureethylester

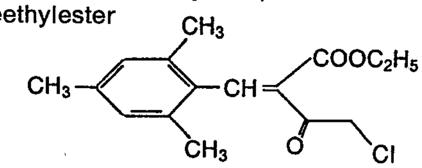

90 g 2,4,6-Trimethylbenzaldehyd, 150 g 4-Chloracetessigester, 2,6 ml Piperidin und 7 ml Eisessig werden in 60 ml Benzol gelöst und 6 Stunden am Wasserabscheider gekocht. Man versetzt die abgekühlte Lösung mit 200 ml Essigester, extrahiert die Lösung je einmal mit gesättigter Natriumhydrogencarbonatlösung, Wasser und 1 m Zitronensäurelösung, trocknet über Magnesiumsulfat und dampft ein. Das Rohprodukt wird im Hochvakuum andestilliert, um es von überschüssigem 4-Chloracetessigester zu befreien. Nach Chromatografie an Kieselgel 60 (Merck, 70–230 mesh) lassen sich 21 g E-Isomeres mit Isopropanol kristallisieren, als Mutterlauge verbleibt ein 3:1-Z:E-Gemisch in Form eines Öls. Gesamt-Ausbeute 39%.

E-Isomeres: Fp. 85°.

IR (Nujol): ~1700, 1610, 1310, 1250, 1175, 1135, 1095, 1030, 970 $cm^{-1}$.

NMR ($CDCl_3$): δ = 8,02[1] s, 6,90[2] s, 4,37[2] q, J = 7 Hz, 4,04[2] s, 2,32[3] s, 2,20[6] s, 1,38[3] t, J = 7 Hz ppm.

| | | | |
|---|---|---|---|
| ber.: | C 65,2 | H 6,5 | Cl 12,0 |
| gef.: | C 65,1 | H 6,8 | Cl 12,0 |

Z-Isomeres:

NMR ($CDCl_3$): δ = 7,98[1] s, 6,84[2] s, 4,56[2] s, 4,06[2] q, J = 7 Hz, 2,32[3] s, 2,20[6] s, 0,91[3] t, J = 7 Hz ppm.

Beispiel 25
2-(2-Aminothiazol-4-yl)-3-(2,4,6-trimethylphenyl)-propensäureethylester (Z-Isomeres)

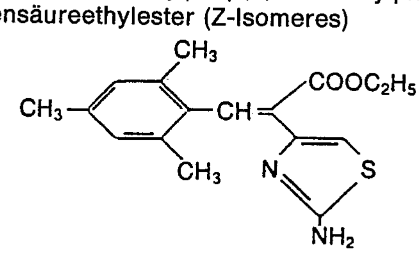

Eine Lösung von 32 g des in Beispiel 24 hergestellten Z/E-Gemisches, 9 g Natriumacetat und 8,8 g Thioharnstoff in 210 ml Tetrahydrofuran und 90 ml Wasser wird 48 Stunden bei Raumtemperatur gerührt. Man zieht das THF ab, stellt die wässrige Phase auf pH 8 und extrahiert dreimal mit Essigester. Die organische Phase wird mit Wasser gewaschen, getrocknet und eingedampft. Kristallisation mit Dioxan liefert 8,5 g des reinen Z-Isomeren, ½ Mol Dioxan enthaltend, Fp. 122–124°. Aus der Mutterlauge kann durch Chromatografie eine zweite Fraktion erhalten werden.

IR (Nujol): 3600, 3210, 1710, 1640, 1540, 1465, 1285, 1230, 1210, 1140, 1110, 1080, 1040 $cm^{-1}$.

NMR (DMF): $\delta$ = 7,56[1] s, 7,30[2] s, 6,91[2] s, 6,30[1] s, 3,99[2] q, J = 7 Hz, 2,23[3] s, 2,16[6] s, 0,86[3] t, J = 7 Hz ppm.

ber. für 1 Mol Produkt + ½ Mol Dioxan:

C 63,2 H 6,7 N 7,8 S 8,9

gef.:

C 63,0 H 6,8 N 7,7 S 9,0

Beispiel 26

2-(2-Aminothiazol-4-yl)-3-(2,4,6-trimethylphenyl)-propensäure (Z-Isomeres)

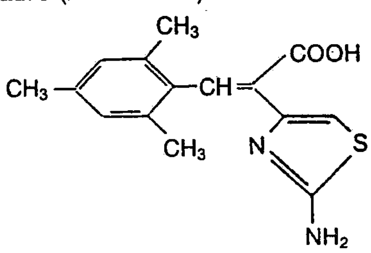

6,1 g des Produkts aus Beispiel 26 werden in einem Gemisch aus 150 ml Dioxan und 45 ml 1,5 n Natronlauge gelöst. Man kocht über Nacht am Rückfluss, zieht dann das Dioxan ab, verdünnt mit 50 ml Wasser und saugt ab. Das Filtrat wird mit verdünnter Salzsäure auf pH 5 gestellt und nach 10 Minuten erneut abgesaugt. Umkristallisation des Rückstands aus Methanol liefert das gewünschte Produkt in 72% Ausbeute. Fp. > 200°.

IR (KBr): 1675, 1565, 1473, 1393, 1307, 1242, 1199, 1171 $cm^{-1}$.

NMR (DMF): $\delta$ = 7,52[1] s, 7,20[2] s, 6,91[2] s, 6,75[1] s, 2,23[9] s ppm.

| | | | | |
|---|---|---|---|---|
| ber.: | C 62,5 | H 5,6 | N 9,7 | S 11,1 |
| gef.: | C 62,0 | H 6,0 | N 9,8 | S 11,2 |

Beispiel 27

2-(2-Dichlorphosphorylaminothiazol-4-yl)-3-(2,4,6-trimethylphenyl)propensäurechlorid (Z-Isomeres)

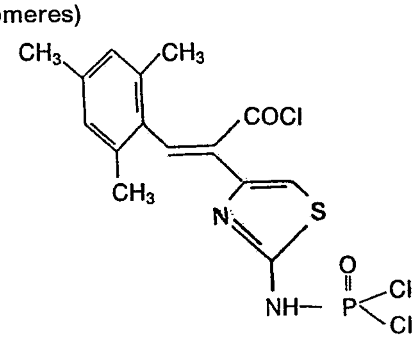

2 g der Carbonsäure aus Beispiel 26 werden unter Stickstoffatmosphäre in 50 ml Dichlormethan suspendiert. Man gibt bei 0° 3 g Phosphorpentachlorid zu und rührt anschliessend 4 Stunden bei Raumtemperatur. Nach Filtration wird das Filtrat eingedampft und das zurückbleibende Öl mit Ether kristallisiert. Ausbeute 1 g. Fp. 161–2° (Zers.).

IR (Nujol): 1760, 1570, 1360, 1310, 1275, 1235, 1175, 1060, 1010, 910 $cm^{-1}$.

NMR ($CDCl_3$): $\delta$ = ~14,5–15,5[1] sehr breit, 7,82[1] s, 6,95[2] s, 6,64[1] d, J = 2 Hz, 2,35[3] s, 2,30[6] s ppm.

Beispiel 28

A) Z-Natrium-6-[2-(2-aminothiazol-4-yl)-2-(2,4,6-trimethylbenzyliden)acetamido]-penam-3-carboxylat

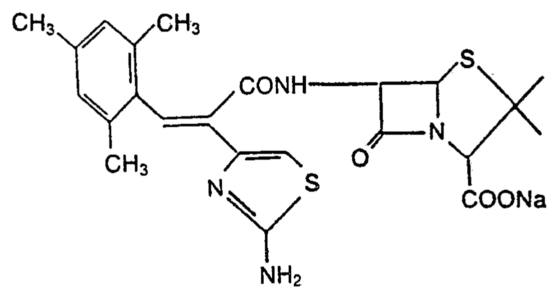

1 g des Produkts aus Beispiel 26 werden gemäss Beispiel 23 umgesetzt. Die bei pH 2,5 gefällte Säure wird in Essigester/Wasser suspendiert und durch Zugabe von 2 n Natronlauge bei pH 7 in Lösung gebracht. Nach Abtrennen des Essigesters wird die wässrige Phase lyophylisiert. Man erhält 0,8 g Natriumsalz, das noch Reste 1-Hydroxybenztriazol enthält.

IR (Nujol): 1750, 1600 $cm^{-1}$.

NMR ($CD_3OD$): $\delta$ = 7,44[1] s, 6,82[2] s, 6,65[1] s, 5,45[1] d, J = 5 Hz, 5,37[1] d, J = 5 Hz, 4,07[1] s, 2,22[9] s, 1,49[3] s, 1,43[3] s.

B) 0,43 g 6-Aminopenicillansäure werden mit Triethylamin bei pH 7–8 in 20 ml 80prozentigem wässrigem THF gelöst. Bei 0° wird das Produkt aus Beispiel 27 eingetragen und der pH dabei durch Zugabe von Triethylamin auf 7,5 gehalten. Man rührt zwei Stunden bei Raumtemperatur nach, zieht dann das THF ab und extrahiert die wässrige Lösung zweimal mit Essigester. Die wässrige Phase wird vom restlichen Essigester befreit und dann mit 2 n Salzsäure auf pH 2,0 gestellt. Man saugt das ausgefallene Produkt ab, suspendiert es in 10 ml Wasser und rührt 3 Stunden bei 50° und pH 3. Es wird erneut abgesaugt und der Rückstand wie beschrieben in das lyophylisierte Natriumsalz überführt. Ausbeute 0,7 g.

Beispiel 29

Z-Natrium-7-[2-(2-aminothiazol-4-yl)-2-(2,4,6-trimethylbenzyliden)acetamido]-3-acetoxymethyl-3-cefem-4-carboxylat

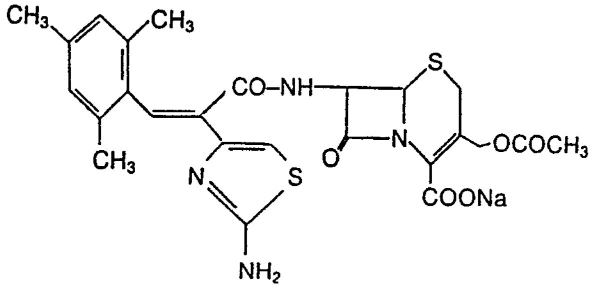

1 g des Produkts aus Beispiel 26 wird gemäss Beispiel 19 umgesetzt. Die bei pH 2,5 gefällte Carbonsäure wird gemäss Beispiel 28 in das Natriumsalz überführt. Ausbeute 1,2 g.

IR (Nujol): 1750, 1600, 1520 $cm^{-1}$.

NMR ($CD_3OD$): $\delta$ = 7,35[1] s, 6,80[2] s, 6,60[1] s, 5,59[1] d, J = 5 Hz, 4,99[1] d, J = 12 Hz, 4,93[1] d, J = 5 Hz, 4,81[1] d, J = 12 Hz, 3,50[1] d, J = 17 Hz, 3,21[1] d, J = 17 Hz, 2,24[6] s, 2,22[3] s, 2,04[3] s ppm.

Beispiel 30

2-(2-Aminothiazol-4-yl)-3-phenylpropensäure-ethylester (Z-Isomeres)

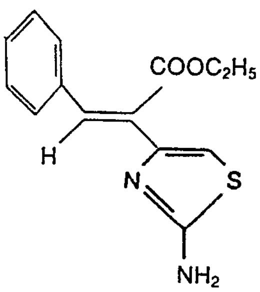

209,2 g 2-Benzyliden-3-oxo-4-chlorbuttersäureethylester (beschrieben in J. Amer. Chem. Soc. 66, 1933 (1944), 63,2 g Thioharnstoff und 104,6 g Natriumacetat werden in einer Mischung aus 1460 ml THF und 630 ml Wasser gelöst. Man rührt über Nacht bei Raumtemperatur und dampft dann das THF ab. Die wässrige Phase wird auf pH 8 gestellt und dreimal mit Essigester extrahiert. Trocknen und Eindampfen der organischen Phase liefert ein zähes Öl, das hauptsächlich aus dem gewünschten Z-Isomeren besteht. Es wird roh weiterverarbeitet. Ausbeute: 222 g.

IR ($CHCl_3$): 1710, 1610, 1525, 1380 $cm^{-1}$.

NMR ($CDCl_3$): $\delta$ = 7,46[1] s, 7,24[5] s, 6,45[1] s, 4,18[2] q, J = 7 Hz, 1,10[3] t, J = 7 Hz ppm.

Beispiel 31

2-(2-Aminothiazol-4-yl)-3-phenylpropensäure (Z-Isomeres)

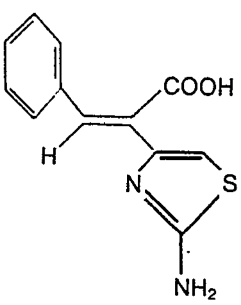

216 g des Rohprodukts aus Beispiel 30 werden in einem Gemisch aus 1,2 l Dioxan und 800 ml 12 proz. Natronlauge gelöst. Man kocht 15 Stunden am Rückfluss, zieht dann das Dioxan ab und stellt mit 2 n Salzsäure auf pH 8. Es wird dreimal mit Essigester extrahiert, die wässrige Lösung auf pH 3–4 gestellt und das ausgefallene Produkt abgesaugt. Durch Umkristallisation aus Methanol lassen sich 20 g des gewünschten Produkts direkt gewinnen, Fp. 138°. Die Mutterlauge besteht aus einem E/Z-Gemisch, welches durch Kristallisation nicht zu trennen ist.

IR (KBr): 1650, 1599, 1579, 1564, 1425, 1344, 1304 $cm^{-1}$.

NMR ($CDCl_3$ + DMSO): $\delta$ = 7,54[1] s, 7,32[5] m, 6,50[1] s ppm.

Beispiel 32

Z-Natrium-6-[2-(2-aminothiazol-4-yl)-2-benzylidenacetamido]-penam-3-carboxylat

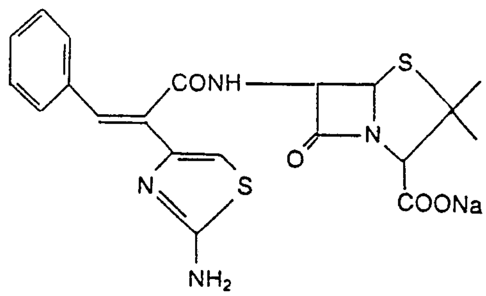

2 g des Produkts aus Beispiel 31 werden gemäss Beispiel 28A umgesetzt. Man erhält 2,6 g Natriumsalz.

IR (Nujol): 1750, 1600 $cm^{-1}$.

NMR ($CD_3OD$): $\delta$ = 7,25[1] s, 7,20[5] m, 6,50[1] s, 5,57[1] d, J = 5 Hz, 5,49[1] d, J = 5 Hz, 4,08[1] s, 1,47[6] s ppm.

Beispiel 33

Z-Natrium-7-[2-(2-aminothiazol-4-yl)-2-benzylidenacetamido]-3-acetoximethyl-3-cefem-4-carboxylat

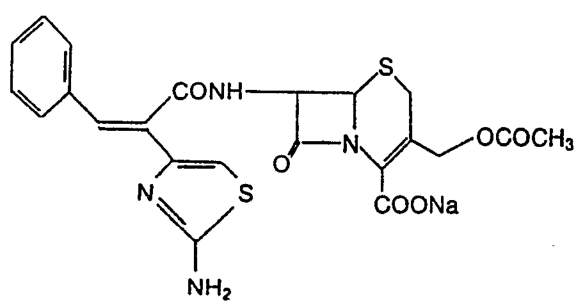

2 g des Produkts aus Beispiel 31 werden gemäss Beispiel 29 umgesetzt. Man erhält 2,8 g Natriumsalz.

IR (Nujol): 1755, 1600 $cm^{-1}$.

NMR ($CD_3OD$): $\delta$ = 7,26[1] s, 7,20[5] m, 6,48[1] s, 5,76[1] d, J = 5 Hz, 5,05[1] d, J = 5 Hz, 4,93[1] d, J = 12,5 Hz, 4,75[1] d, J = 12,5 Hz, 3,52[1] d, J = 18 Hz, 3,24[1] d, J = 18 Hz, 1,98[3] s ppm.

Beispiel 34
E-6-[2-(2-Aminothiazol-4-yl)-2-(2-furyliden)acetamido]penam-3-carboxylat

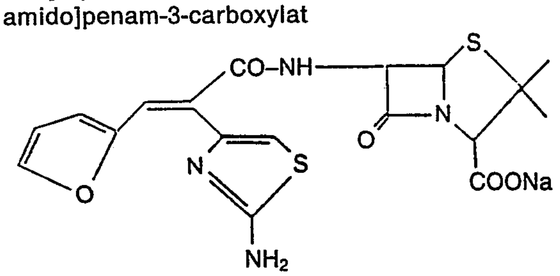

4 g des nach Beispiel 12 hergestellten Produkts werden gemäss Beispiel 28A umgesetzt. Man erhält 3,5 g Natriumsalz.
IR (Nujol): 1750, 1595 $cm^{-1}$.
NMR ($CD_3OD$): $\delta$ = 7,62[1] s, 7,56[1] s breit, 6,64[1] s, 6,48[2] m, 5,62[2] s, 4,22[1] s, 1,55[3] s, 1,53[3] s ppm.

Beispiel 35
2-(2-Aminothiazol-4-yl)-3-(2-furyl)-propensäure-ethylester (Z-Isomeres)

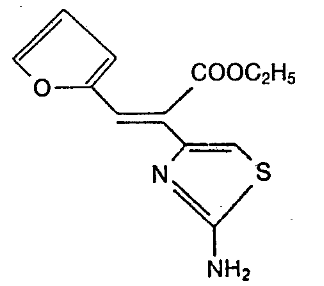

200 g des nach Beispiel 10 hergestellten Isomerengemisches, 68 g Thioharnstoff und 68 g Natriumacetat werden in einem Gemisch aus 1 l THF und 250 ml Wasser gelöst. Man rührt 24 Stunden bei Raumtemperatur, zieht das THF ab und stellt die wässrige Phase auf pH 8. Es wird dreimal mit Essigester extrahiert, die vereinigten Essigester-Phasen werden einmal mit Wasser gewaschen, über Magnesiumsulfat getrocknet und eingeengt, wodurch eine kleine Menge des E-Isomeren (4,7 g) kristallisiert werden kann. Die Mutterlauge wird vollständig eingedampft und das zurückbleibende Öl mit Ethanol kristallisiert. Man erhält 106 g des reinen Z-Isomeren, Fp. 109–110°.
IR (Nujol): 1700, 1635, 1590, 1520, 1350, 1230, 1190, 1140, 1020 $cm^{-1}$.
NMR (DMF): $\delta$ = 7,65[1] m, 7,18[3] s verbreitert, 6,50[3] m, 4,35[2] q, J = 7 Hz, 1,30[3] t, J = 7 Hz ppm.

| | | | | |
|---|---|---|---|---|
| ber. | C 54,5 | H 4,6 | N 10,6 | S 12,1 |
| gef. | C 53,9 | H 4,6 | N 10,5 | S 11,2 |

Beispiel 36
2-(2-Aminothiazol-4-yl)-3-(2-furyl)-propensäure (Z-Isomeres)

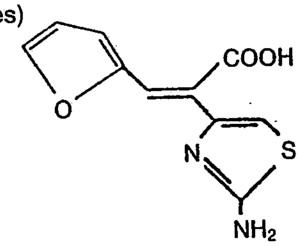

Eine Lösung von 61 g des nach Beispiel 35 hergestellten Produkts in 1 l Dioxan wird mit 300 ml 2n Natronlauge versetzt und 7 Stunden am Rückfluss gekocht. Man zieht das Dioxan ab, stellt die wässrige Lösung mit 2n Salzsäure auf pH 9 und extrahiert dreimal mit Essigester. Anschliessend stellt man auf pH 2,0 und saugt ab. Umkristallisation aus Ethanol liefert 41 g des Produkts, Fp. 143° (Zers.).
IR (KBr): 1660, 1635, 1612, 1575, 1468, 1421, 1308 $cm^{-1}$.
NMR (DMSO): $\delta$ = 7,62[1] d, J = 2 Hz, 6,91[1] s, 6,53[2] m, 6,42[1] s ppm.

Beispiel 37
2-(2-Aminothiazol-4-yl)-3-(2-furyl)-propensäurechloridhydrochlorid (Z-Isomeres)

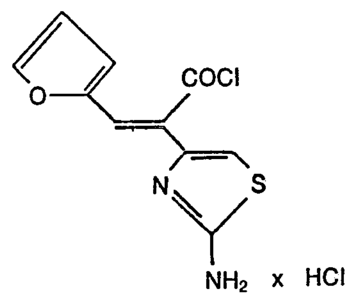

10 g des nach Beispiel 36 hergestellten getrockneten Produkts werden unter Stickstoffatmosphäre in 120 ml abs. Methylenchlorid suspendiert. Man gibt bei 0° 17,5 g Phosphorpentachlorid zu, lässt auf Raumtemperatur kommen und rührt 4,5 Stunden nach. Es wird vom Produkt abgesaugt, Ausbeute 6,4 g, Fp. 123° (Z.).
IR (Nujol): 1779, 1622, 1295 $cm^{-1}$.
NMR (DMF): $\delta$ = 12,7[3] s sehr breit, 7,83[1] d, J = 2 Hz, 7,46[1] s, 6,96[1] s, 6,91[1] d, J = 4 Hz, 6,64[1] dd, J = 2 Hz, J = 4 Hz ppm.
MS: 192, 150

Beispiel 38
Z-Natrium-7-[2-(2-aminothiazol-4-yl)-2-(2-furyliden)-acetamido]-3-acetoxymethyl-3-cefem-4-carboxylat

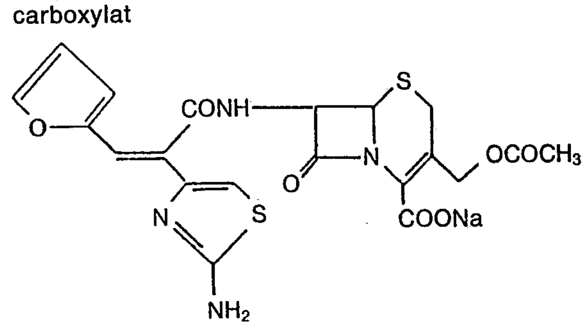

Zu einer Lösung von 0,8 g 7-Aminocefalosporansäure und 1,3 ml Triethylamin in 100 ml abs. Methylenchlorid wird bei 0° 1 g des Produkts aus Beispiel 37 gegeben. Man rührt 30 Minuten bei 0° und 4 Stunden bei Raumtemperatur nach, giesst dann auf Wasser und stellt auf pH 6,5 ein. Nach Abziehen des organischen Lösungsmittels wird die wässrige Phase dreimal mit Essigester extrahiert und dann auf pH 2,5 gestellt. Man saugt ab

und überführt den Rückstand, wie in Beispiel 28 beschrieben, in das Natriumsalz. Ausbeute 1,1 g.

IR (Nujol): 1750, 1600, 1520, 1325, 1232, 1020 $cm^{-1}$.

NMR ($CD_3OD$): δ = 7,60[1] d, J = 1,5 Hz, 7,15[1] s, 6,61[1] d, J = 4 Hz, 6,56[[1] s, 6,51[1] dd, J = 4 Hz, J = 1,5 Hz, 5,98[1] d, J = 5Hz, 5,21[1] d, J = 5 Hz, 5,05[1] d, J = 13 Hz, 4,88[1] d, J = 13 Hz, 3,66[1] d, J = 18 Hz, 3,38[1] d, J = 18 Hz, 2,07[3] s ppm.

Beispiel 39

Z-Natrium-6-[2-(2-aminothiazol-4-yl)-2-(2-furyliden)acetamido]-penam-4-carboxylat

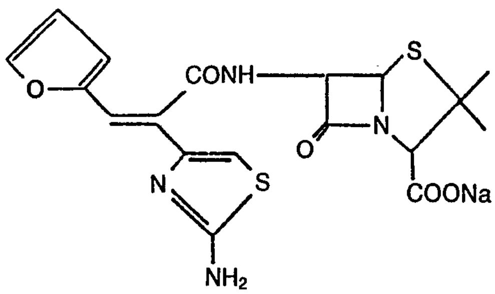

2 g des Säurechlorids aus Beispiel 37 werden analog zu Beispiel 38 mit 1,5 g 6-Aminopenicillansäure umgesetzt. Man erhält 2,4 g Natriumsalz.

IR (Nujol): 3300, 1750, 1600, 1510, 1310 $cm^{-1}$.

NMR ($CD_3OD$): δ = 7,57[1] d, J = 2 Hz, 7,13[[1] s, 6,56[1] s, 6,56[1] d, J = 3 Hz, 6,49[1] dd, J = 2 Hz, J = 3 Hz, 4,79[1] d, J = 5 Hz, 5,69[1] d, J = 5 Hz, 4,24[1] s, 1,26[3] s, 1,22[3] s ppm.

Beispiel 40

2-(2-Dichlorphosphorylaminothiazol-4-yl)-3-(2,6-dichlorphenyl)propensäurechlorid (Z-Isomeres)

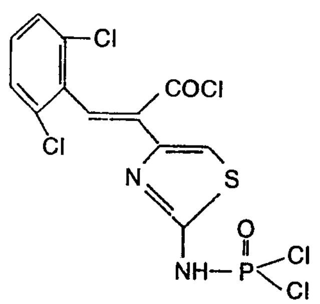

In eine Suspension von 20 g des nach Beispiel 6 hergestellten Produkts in 200 ml abs. Methylenchlorid werden unter Stickstoffatmosphäre bei 0° 26,2 g Phosphorpentachlorid portionsweise eingetragen. Man rührt nach, bis eine klare Lösung entstanden ist und dampft diese dann ein. Der Rückstand wird mit Ether verrührt, das Produkt abgesaugt, gut mit Ether gewaschen und aus Acetonitril umkristallisiert. Ausbeute 10 g, Fp. 174–6° (Zers.).

IR (Nujol): 1760, 1570, 1545, 1235, 1000 $cm^{-1}$.

NMR (Aceton-$d_6$): δ = 7,80[1] s, 7,58[3] s, 7,43[[1] d, J = 2 Hz ppm.

MS: 448, 413, 377, 117, 82, 66, 47, 36.

ber.:

C 32,0 H 1,3 N 6,2 S 7,1 Cl 39,4 P 6,9

gef.:

C 32,1 H 1,7 N 5,8 S 7,4 Cl 39,2 P 7,2

Beispiel 41

Z-6-[2-(2-Dihydroxyphosphorylaminothiazol-4-yl)-2-(2,6-dichlorbenzyliden)acetamido]penam-3-carbonsäure

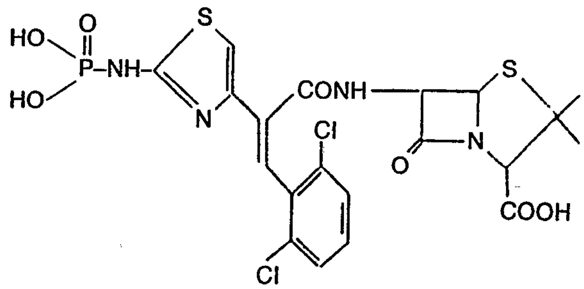

3 g 6-Aminopenicillansäure werden mit einem Equivalent Triethylamin in 100 ml 80prozentigem wässrigen THF in Lösung gebracht. Man kühlt auf 0° und trägt 5 g des Produkts aus Beispiel 40 ein, wobei die pH der Lösung durch Zugabe von Triethylamin auf 7,5 gehalten wird. Man rührt 30 Minuten nach, zieht dann das THF und stellt die wässrige Lösung mit 2 n Salzsäure auf pH 1,8. Es wird eine Stunde bei diesem pH gerührt, dann mit 2 n Natronlauge auf pH 6,5 gestellt und dreimal mit Essigester extrahiert. Die wässrige Phase wird erneut auf pH 1,8 gestellt und das Produkt abgesaugt, mit Wasser gewaschen und getrocknet. Es enthält ein Mol Triethylamin. Ausbeute 7,5 g.

IR (Nujol): 1775, 1740, 1665, 1570, 1550, 1495, 1290, 1080 $cm^{-1}$.

NMR ($CD_3OD$/DMF): δ = 7,50[4] m, 7,25[1] s, 5,71[1] d, J = 4 Hz, 5,62[1] d, J = 4 Hz, 4,45[1] s, 1,65[3] s, 1,58[3] s ppm.

Beispiel 42

Z-Natrium-6-[2-(2-aminothiazol-4-yl)-2-(2,6-dichlorbenzyliden)acetamido]penam-3-carboxylat

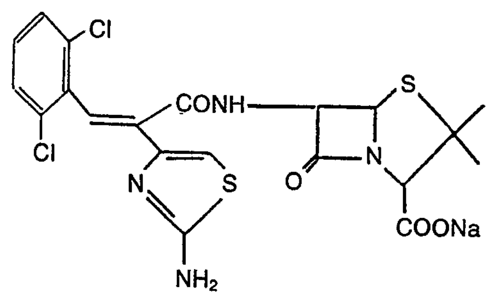

Eine Suspension von 4 g des Produkts aus Beispiel 41 in 20 ml Wasser wird 3 Stunden bei pH 3 unter Rühren auf 50° erwärmt. Man saugt ab, wäscht den Rückstand mit Wasser und überführt ihn gemäss Beispiel 28 in das Natriumsalz. Ausbeute 2,4 g.

IR (Nujol): 1755, 1600, 1500, 1300, 1090 $cm^{-1}$.

NMR ($CD_3OD$): δ = 7,45[3] m, 7,36[1] s, 6,85[1] s, 5,55[1] d, J = 4 Hz, 5,51[[1] d, J = 4 Hz, 4,20[1] s, 1,40[3] s, 1,38[3] s ppm.

Beispiel 43
2-(2-Trifluormethylbenzyliden)-3-oxo-4-chlor-buttersäureethylester

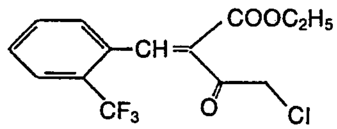

226,2 g 2-Trifluormethylbenzaldehyd, 235 g 4-Chloracetessigsäureethylester, 4,2 ml Eisessig und 2,1 ml Piperidin werden in 500 ml Benzol gelöst und 8 Stunden am Wasserabscheider gekocht. Man gibt 1 l Essigester zu, extrahiert zweimal mit Natriumbicarbonat, einmal mit Zitronensäure und noch einmal mit Wasser, trocknet und dampft ein. Das Rohprodukt wird im Hochvakuum bis 130° andestilliert, als Rückstand verbleibt das Produkt 2:1-Isomerengemisch, gaschromatographisch rein. Ausbeute 234 g.

IR (Film): 1720, 1620, 1575, 1490, 1450, 1400, 1375, 1320, 1300, 1260, 1175, 1130, 1062, 1040 $cm^{-1}$.

NMR ($CDCl_3$): δ = 8,20[1] m, 7,3–7,8[4] m, 4,59/4,40[2] s, 4,38/4,10[2] q, J = 7 Hz, 1,32/1,00[3] t, J = 7 Hz ppm.

| | | | | |
|---|---|---|---|---|
| ber.: | C 52,4 | H 3,8 | Cl 11,1 | F 17,8 |
| gef.: | C 51,5 | H 3,7 | Cl 11,3 | F 17,3 |

Beispiel 44
2-(2-Aminothiazol-4-yl)-3-(2-trifluormethylphenyl)-propensäureethylester

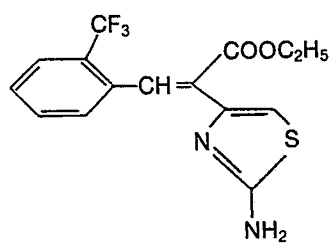

Eine Lösung von 234 g des Produkts aus Beispiel 43 und 55,5 g Thioharnstoff in 500 ml Ethanol wird 3 Stunden bei 50° gerührt und dann eingedampft. Den Rückstand nimmt man in Wasser/Essigester auf, stellt auf pH 8 und extrahiert die wässrige Phase dreimal mit Essigester. Die vereinigten Essigesterextrakte werden einmal mit Wasser gewaschen, über Magnesiumsulfat getrocknet und auf etwa 300 ml eingeengt. Nach Stehen über Nacht saugt man ab und wäscht den Rückstand mit Ether. Man erhält so 44,8 g des E-Isomeren. Fp. 145–7°.

Aus der eingedampften Mutterlauge lassen sich durch fraktionierte Kristallisation aus Toluol 68 g des reinen Z-Isomeren gewinnen. Fp. 97–8°.

E-Isomeres:

IR (Nujol): 3350, 1690, 1635, 1625, 1520, 1310, 1260, 1150, 1120, 1035 $cm^{-1}$.

NMR ($CDCl_3$): δ = 8,0[1] m, 7,0–7,8[4] m, 6,16[1] s, 5,24[2] s breit, 4,30[2] q, J = 7 Hz, 1,30[[3] t, J = 7 Hz ppm.

| | | | | | |
|---|---|---|---|---|---|
| ber.: | C 52,6 | H 3,8 | N 8,2 | S 9,4 | F 16,6 |
| gef.: | C 51,6 | H 3,9 | N 7,9 | S 9,3 | F 16,7 |

Z-Isomeres:

IR (Nujol): 3350, 3220, 3100, 1700, 1623, 1530, 1350, 1310, 1260, 1230, 1170, 1110, 1060, 1025 $cm^{-1}$.

NMR (DMSO): δ = 7,3–7,9[5] m, 7,23[2] s breit, 6,65[1] s, 4,06[2] q, J = 7 Hz, 0,95[3] t, J = 7 Hz ppm.

| | | | | | |
|---|---|---|---|---|---|
| ber.: | C 52,6 | H 3,8 | N 8,2 | S 9,4 | F 16,6 |
| gef.: | C 52,1 | H 4,1 | N 7,9 | S 9,5 | F 17,2 |

Beispiel 45
2-(2-Aminothiazol-4-yl)-3-(2-trifluormethylphenyl)-propensäure (E-Isomeres)

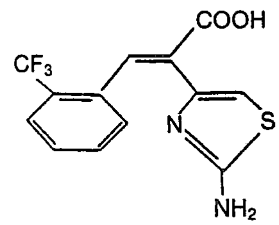

44 g des nach Beispiel 44 hergestellten E-Isomeren werden gemäss Beispiel 15 umgesetzt. Das Produkt wird aus Methanol umkristallisiert. Ausbeute 33,5 g, Fp. 222–3 (Zers.).

IR (Nujol): 1650 (Schulter), 1620, 1590, 1520, 1315, 1180, 1120, 1095, 1060, 1030 $cm^{-1}$.

NMR (DMSO): δ = 7,1–8,0[5] m, 7,06[2] s breit, 6,23[1] s ppm.

| | | | | | |
|---|---|---|---|---|---|
| ber.: | C 49,7 | H 2,9 | N 8,9 | S 10,2 | F 18,1 |
| gef.: | C 49,1 | H 3,2 | N 8,9 | S 10,0 | F 18,3 |

Beispiel 46
2-(2-Aminothiazol-4-yl)-3-(2-trifluormethylphenyl)-propensäure (Z-Isomeres)

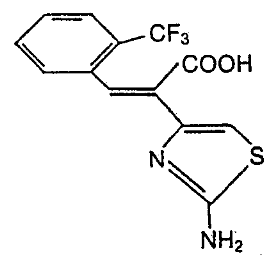

66,5 g des nach Beispiel 44 hergestellten Z-Isomeren werden gemäss Beispiel 36 umgesetzt. Nach Umkristallisation aus Methanol erhält man 43,4 g Produkt, Fp. 205–7° (Zers.).

IR (Nujol): 1625, 1400, 1310, 1260, 1170, 1108 $cm^{-1}$.

NMR (DMSO): δ = 7,3–7,8[4] m, 7,53[1] s, 7,14[[2] s breit, 6,57[1] s ppm.

| | | | | | |
|---|---|---|---|---|---|
| ber.: | C 49,7 | H 2,9 | N 8,9 | S 10,2 | F 18,1 |
| gef.: | C 49,5 | H 3,5 | N 8,6 | S 10,0 | F 18,2 |

Beispiel 47
Z-6-[2-(2-Aminothiazol-4-yl)-2-(2-trifluormethyl-benzyliden)acetamido]penam-3-carbonsäure

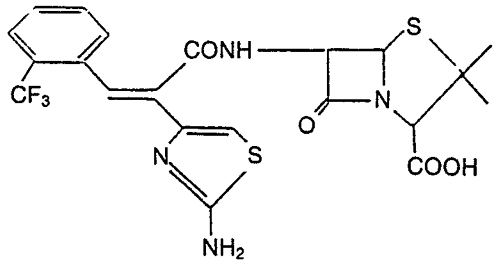

4,7 g des nach Beispiel 46 hergestellten Produkts werden gemäss Beispiel 23 umgesetzt, man erhält 4,2 g Produkt.

IR (Nujol): 1750, 1715, 1650, 1600, 1310 $cm^{-1}$.

NMR ($CD_3OD$): δ = 7,3–7,8[5] m, 6,67[1] s, 5,56[1] d, J = 4 Hz, 5,48[1] d, J = 4 Hz, 4,16[1] s, 1,50[3] s, 1,47[3] s ppm.

Beispiel 48

Z-Natrium-7-[2-(2-aminothiazol-4-yl)2-(2-trifluormethylbenzyliden)acetamido]-3-acetoximethyl-3-cefem-4-carboxylat

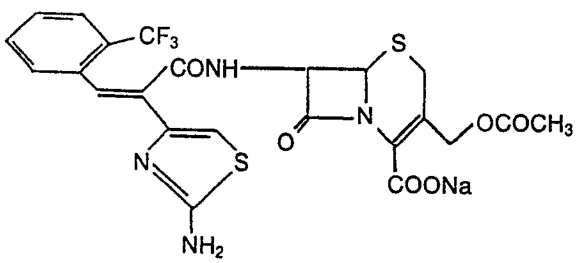

4,7 g des Produkts aus Beispiel 46 werden gemäss Beispiel 29 umgesetzt, man erhält 5,2 g Natriumsalz.

IR (Nujol): 1750, 1600, 1520, 1310 $cm^{-1}$.

NMR ($CD_3OD$): δ = 7,7[3] m, 7,53[1] t, J = 6 Hz, 7,41[1] t, J = 6 Hz, 6,65[1] s, 5,74[1] d, J = 4 Hz, 5,02[1] d, J = 4 Hz, 4,99]1] d, J = 12 Hz, 4,85[1] d, J = 12 Hz, 3,52[1] d, J = 18 Hz, 3,26[1] d, J = 18 Hz, 2,00[3] s ppm.

Beispiel 49

E-Natrium-7-[2-(2-aminothiazol-4-yl)-2-(2-trifluormethylbenzyliden)acetamido]-3-acetoximethyl-3-cefem-4-carboxylat

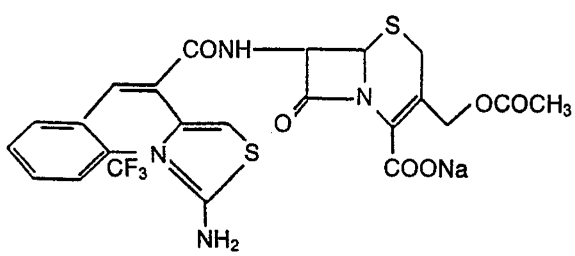

4,7 g des nach Beispiel 45 hergestellten Produkts werden gemäss Beispiel 29 umgesetzt, man erhält 4,5 g Natriumsalz.

IR (Nujol): 1750, 1600, 1310, 1100 $cm^{-1}$.

NMR ($CD_3OD$): δ = 7,95[1] s, 7,68[1] m, 7,40[2] m, 7,18[1] m, 5,91[1] s, 5,87[1] d, J = 4 Hz, 5,11[1] d, J = 4 Hz, 5,03[1] d, J = 12 Hz, 4,87[1] d, J = 12 Hz, 3,60[1] d, J = 17 Hz, 3,35[1] d, J = 17 Hz, 2,02[3] s ppm.

Beispiel 50

2-(2,3,6-Trichlorbenzyliden)-3-oxo-4-chlorbuttersäureethylester

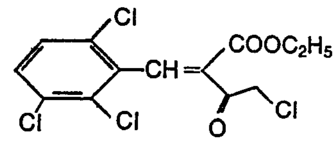

210 g 2,3,6-Trichlorbenzaldehyd werden gemäss Beispiel 43 mit 4-Chloracetessigester umgesetzt. Man erhält 213 g eines 3:1-Isomerengemisches als öliges Rohprodukt.

NMR (DMSO): δ = 7,5–8,0[3] m, 5,02/4,80[2] s, 4,10/4,30[2] q, J = 7 Hz, 1,33/0,96[3] t, J = 7 Hz ppm.

Beispiel 51

2-(2-Aminothiazol-4-yl)-3-(2,3,6-trichlorphenyl)-propensäureethylesterhydrochlorid

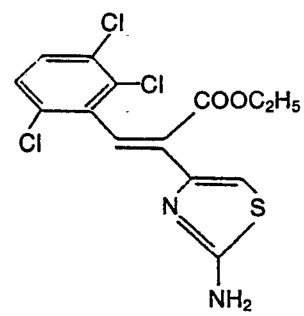

x HCl

213 g des Produkts aus Beispiel 50 und 30 g Thioharnstoff werden, gelöst in 500 ml Ethanol, 15 Stunden bei 15° gerührt. Man dampft ein und kristallisiert den Rückstand mit Aceton. Nach Umkristallisation aus Aceton erhält man so 35 g des Z-Isomeren, Fp. 178°.

Durch Eindampfen der Mutterlauge erhält man 166 g E/Z-Gemisch als zähes Öl, das nach Überführung in die freie Base chromatografisch getrennt werden kann (Laufmittel Methylenchlorid/Methanol = 95/5).

Z-Isomeres: (Hydrochlorid)

IR (Nujol): 1700, 1620, 1550, 1295, 1235, 1175, 1140, 1030 $cm^{-1}$.

NMR (DMSO): δ = 9,10[3] s breit, 7,71[1] s, 7,76[1] d, J = 9 Hz, 7,60[1] d, J = 9 Hz, 7,20[1] s, 4,05[2] q, J = 7 Hz, 0,90[3] t, J = 7 Hz ppm.

| | | | | | |
|---|---|---|---|---|---|
| ber.: | C 40,6 | H 2,9 | N 6,8 | S 7,7 | Cl 34,2 |
| gef.: | C 40,7 | H 2,9 | N 6,6 | S 7,5 | Cl 33,0 |

E-Isomeres: (freie Base)

Fp. 172°.

IR (Nujol): 1690, 1625, 1525, 1275, 1245 $cm^{-1}$.

NMR (DMSO): δ = 7,62[[1] d, J = 9 Hz, 7,48[1] d, J = 9 Hz, 7,26[1] s, 6,72[2] s breit, 6,48[1] s, 4,28[2] q, J = 7 Hz, 1,30[3] t, J = 7 Hz ppm.

Beispiel 52

2-(2-Aminothiazol-4-yl-3-(2,3,6-trichlorphenyl)-propensäure (Z-Isomeres)

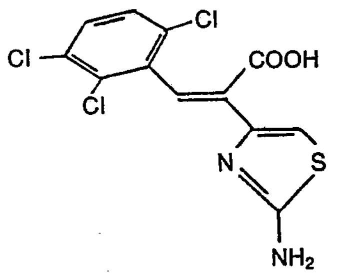

25 g des Z-Isomeren aus Beispiel 51 und 100 ml 2 n NaOH werden, in 500 ml Dioxan gelöst, 5 Stunden am Rückfluss gekocht. Aufarbeitung gemäss Beispiel 36 liefert 18,5 g Produkt, Fp. >210°.

IR (Nujol): 1600, 1565, 1300, 1225, 1170, 1090 $cm^{-1}$.

NMR (DMSO): δ = 7,63[1] d, J = 9 Hz, 7,50[1] d, J = 9 Hz, 7,46[1] s, 6,92[1] s ppm.

Beispiel 53

2-(2-Dichlorphosphorylaminothiazol-4-yl)-3-(2,3,6-trichlorphenyl)propensäurechlorid (Z-Isomeres)

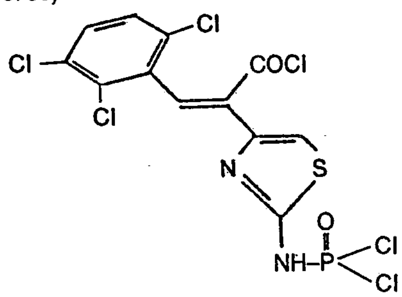

3,5 g des Produkts aus Beispiel 52 werden gemäss Beispiel 40 umgesetzt. Man erhält 2,8 g kristallines Produkt aus Ether, Fp. 200° (Zers.).

NMR ($CDCl_3$): δ = 7,70[1] s, 7,53[1] s, 7,48[1] s, 7,36[1] s, 6,98[1] d, J = 2 Hz, ppm.

Beispiel 54

2-(2-Aminothiazol-4-yl)-3-(2,6-dichlorphenylpropensäurechlorid-hydrochlorid (Z-Isomeres)

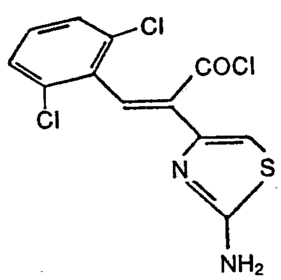

x HCl

x ½ $CH_2Cl_2$

Zu einer Suspension von 1 g des Produkts aus Beispiel 6 in 30 ml Methylenchlorid gibt man 30 µl Wasser und anschliessend bei 0° 1,3 g Phosphorpentachlorid. Es wird 3 Stunden bei Raumtemperatur gerührt und das Produkt dann abgesaugt; mit Methylenchlorid gewaschen und getrocknet. Ausbeute 1,1 g, Fp. 234° (Zers.).

IR (Nujol): 3210, 1760, 1630, 1570, 1550, 1300, 1255, 1195, 1180, 1090, 1030 $cm^{-1}$.

NMR (THF-$D_8$): δ = 7,86[1] s, 7,36[3] m, 6,85[1] s ppm.

| | | | | | |
|---|---|---|---|---|---|
| ber.: | C 36,3 | H 2,2 | N 6,8 | S 7,7 | Cl 43,0 |
| gef.: | C 35,9 | H 2,0 | N 6,7 | S 7,7 | Cl 41,1 |

Beispiel 55

Z-Natrium-7-[2-(2-aminothiazol-4-yl)-2-(2,3,6-trichlorbenzyliden)acetamido]-3-acetoxymethyl-3-cefem-4-carboxylat

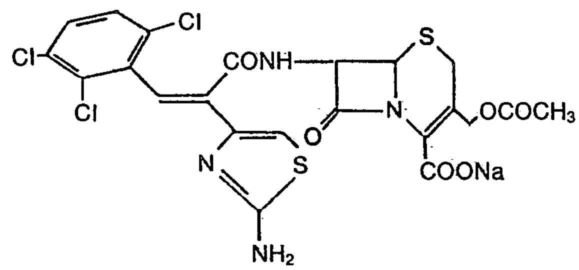

4 g des Produkts aus Beispiel 52 werden gemäss Beispiel 29 umgesetzt, man erhält 6 g Natriumsalz.

IR (Nujol): 1755, 1650, 1600, 1510, 1230 $cm^{-1}$.

NMR ($CD_3OD$): δ = 7,44[1] d, J = 8 Hz, 7,36[1] d, J = 8 Hz, 7,24[1] s, 6,76[1] s, 5,65[1] d, J = 5 Hz, 4,98[1] d, J = 12 Hz, 4,96[[1] d, J = 5 Hz, 4,80[1] d, J = 12 Hz, 3,53[1] d, J = 18 Hz, 3,25[1] d, J = 18 Hz, 2,02[3] s ppm.

Beispiel 56

Z-Natrium-6-[2-(2-aminothiazol-4-yl)-2-(2,3,6-trichlorbenzyliden)acetamido]penam-4-carboxylat

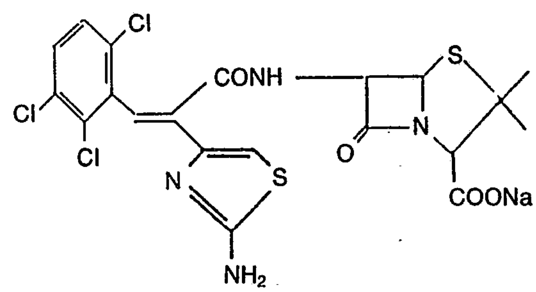

4 g des Produkts aus Beispiel 52 werden gemäss Beispiel 28 umgesetzt, man erhält 3,3 g Natriumsalz.

IR (Nujol): 1750, 1600, 1190, 1170, 1095 $cm^{-1}$.

NMR ($CD_3Od$): δ = 7,45[1] d, J = 8 Hz, 7,37[1] d, J = 8 Hz, 7,28[1] s, 6,79[1] s, 5,47[2] m, 4,15[1] s, 1,53[3] s, 1,51[3] s ppm.

Beispiel 57

2-(2-tert.-Butoxycarbonylaminothiazol-4-yl)-4-oxo-penten-2-säureethylester

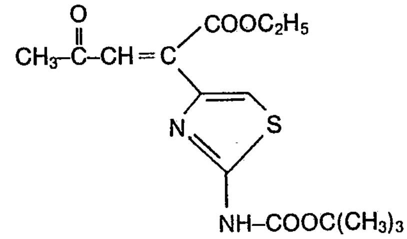

In einem ausgeheizten Kolben werden unter Stickstoff 3,5 g Natriumhydrid in 100 ml abs. Dimethoxyethan (DME) suspendiert. 20 g Diethyl-2-oxo-propylphosphonat werden, gelöst in 100 ml DME, bei Raumtemperatur zugetropft. Man rührt eine Stunde nach und tropft dann eine Lösung von 30 g 2-(2-tert.-Butoxycarbonylaminothiazol-4-yl)-glyoxylsäureethylester in 100 ml DME zu. Nach Rühren über Nacht wird mit Eis und 1n Zitronensäurelösung hydrolysiert und auf pH 6 eingestellt. Man zieht das DME ab und extrahiert die wässrige Phase dreimal mit Essigester. Die vereinigten Essigester-Phasen werden getrocknet und eingeengt. Das zurückbleibende Öl wird mit Ether-Petrolether kristallisiert und dieses Rohprodukt (Ausbeute 62%, Fp.: 107–8°) aus Cyclohexan umkristallisiert. Ausbeute: 44%, Fp.: 108–110°. Man erhält ein reines Isomeres.

IR (KBr): 3284 (breit), 3085, 1717, 1700 (Schulter), 1686, 1591, 1550, 1246, 1150 $cm^{-1}$.

NMR ($CDCl_3$): $\delta$ = 7,10[1] s, 6,96[1] s, 4,42[2] q, J = 7 Hz, 2,28[3] s, 1,54[9] s, 1,38[3] t, J = 7 Hz ppm.

| | | | | |
|---|---|---|---|---|
| ber.: | C 52,9 | H 5,9 | N 8,2 | S 9,4 |
| gef.: | C 52,1 | H 5,9 | N 7,4 | S 8,5 |

Beispiel 58

2-(2-tert.-Butoxycarbonylaminothiazol-4-yl)-4-hydroxy-4-methylbutenolid

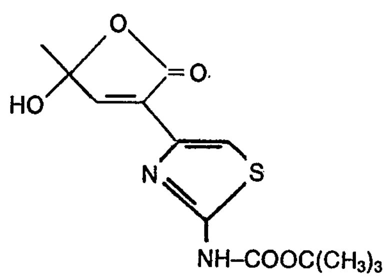

Eine Suspension von 15 g des in Beispiel 57 hergestellten Produkts in einem Gemisch aus 100 ml Methanol und 20 ml Wasser wird mit 2n Natronlauge auf pH 13 eingestellt und bis zur pH-Konstanz bei Raumtemperatur gerührt (2–3 Stunden). Man stellt auf pH 7, zieht das Methanol ab, saugt ab und wäscht das Filtrat zweimal mit Essigester. Die wässrige Phase wird dann auf pH 1,8 eingestellt und dreimal mit Essigester extrahiert. Die vereinigten Essigesterphasen werden getrocknet und eingedampft, man gewinnt das Produkt als gelben Hartschaum. Ausbeute: 88%.

IR (KBr): 3399, 1769, 1727, 1543, 1443, 1371, 1208, 1154 $cm^{-1}$.

NMR ($CDCl_3$): $\delta$ = 7,70[1] s, 7,35[1] s, 6,80[2] s sehr breit, 1,76[3] s, 1,57[9] s ppm.

MS: 312, 256, 212, 197, 195, 169, 151, 125, 57.

| | | | | |
|---|---|---|---|---|
| ber.: | C 50,0 | H 5,2 | N 9,0 | S 10,3 |
| gef.: | C 49,2 | H 5,3 | N 8,5 | S 9,9 |

Beispiel 59

Triethylammonium-2-(2-tert.-butoxycarbonylaminothiazol-4-yl)-3-oxo-pentenoat

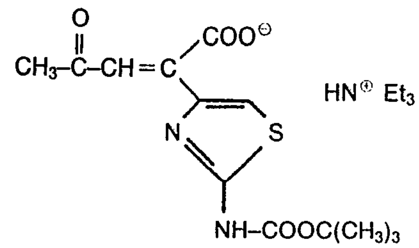

Das in Beispiel 58 hergestellte Produkt wird in Essigester gelöst und die Lösung mit Wasser versetzt. Man stellt mit Triethylamin auf pH 7 ein, trennt den Essigester ab und lyophylisiert die wässrige Phase.

IR (KBr): 1608, 1527, 1370, 1239 (alle breit), 1140 $cm^{-1}$.

NMR ($D_2O$): $\delta$ = 7,34[1] s, 6,77[1] s, 3,21[6] q, J = 7 Hz, 2,33[3] s, 1,52[9] s, 1,29[9] t, J = 7 Hz ppm.

Beispiel 60

2-(2-tert.-Butoxycarbonylaminothiazol-4-yl)-4-methoxyiminopenten-2-säure

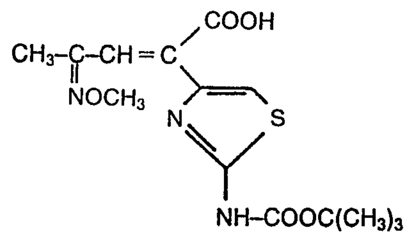

Eine Lösung von 12,75 g des Produkts aus Beispiel 58, 3,6 g Hydroxylaminmethyletherhydrochlorid und 12,5 ml Triethylamin in 150 ml Ethanol wird 24 Stunden bei Raumtemperatur gerührt. Man zieht das Ethanol ab, nimmt den Rückstand in Essigester auf und extrahiert dreimal mit 2n Salzsäure und einmal mit Wasser. Nach Trocknen und Eindampfen wird das zurückbleibende Öl aus Acetonitril oder Tetrachlormethan kristallisiert. Ausbeute 7,7 g, Fp.: 165° (Zers.).

IR (Nujol): 3150, 1700, 1550, 1300, 1250, 1180, 1155, 1080, 1055 $cm^{-1}$.

NMR (DMSO): $\delta$ = 7,08[1] s, 6,83[1] s, 3,87[3] s, 1,98[3] s, 1,50[9] s ppm.

MS: 341, 296, 285, 241, 222

| | | | | |
|---|---|---|---|---|
| ber. (x⅓ $CCl_4$): | C 43,8 | H 4,8 | N 10,7 | S 8,1 |
| gef.: | C 44,3 | H 5,1 | N 10,4 | S 8,0 |

Beispiel 61

Tert.-Butyl-7-[2-(2-tert.-butoxycarbonylamino-thiazol-4-yl)-4-methoxyimino-pent-2-enoyl]amino-3-acetoxymethyl-3-cefem-4-carboxylat

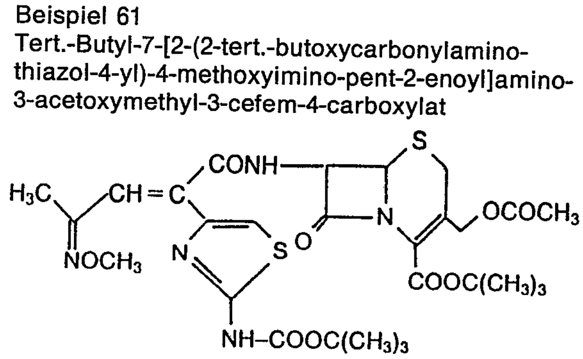

Eine Lösung von 1 g des Produkts aus Beispiel 60 in 30 ml abs. Tetrahydrofuran wird mit 0,4 g 1-Hydroxybenztriazol und 0,6 g DCC versetzt. Man rührt 5 Stunden bei Raumtemperatur, gibt dann

eine Lösung von 1 g 7-Aminocefalosporansäure-tert.-butylester in 20 ml Tetrahydrofuran hinzu und rührt über Nacht bei Raumtemperatur. Es wird vom Dicyclohexylharnstoff abgesaugt, eingedampft und der Rückstand in Essigester gelöst. Man extrahiert dreimal mit Natriumhydrogencarbonatlösung und einmal mit Wasser, trocknet und dampft ein. Verreiben des Rückstandes mit Ether, Filtration und Eindampfen des Filtrats liefert 1,2 g eines Rohprodukts, das durch Chromatographie an Kieselgel (Laufmittel Toluol/Essigester 4:1) gereinigt wird. Ausbeute 320 mg.

IR (KBr): 3284, 2979, 2932, 1787, 1725, 1697, 1551, 1453, 1370, 1245, 1154, 1105, 1052 $cm^{-1}$.

NMR ($CDCl_3$): δ = 8,70[1] s breit, 7,46[1] d, J = 9 Hz, 7,20[1] s, 7,03[1] s, 5,89[1] dd, J = 9 Hz, J = 5 Hz, 5,10[1] d, J = 13 Hz, 5,01[1] d, J = 5 Hz, 4,79[1] d, J = 13 Hz, 3,88[3] s, 3,55[1] d, J = 18 Hz, 3,36[1] d, J = 18 Hz, 2,08[3] s, 2,07[3] s, 1,52[18] s ppm.

Beispiel 62
2-(2,4,5-Trimethoxybenzyliden)-3-oxo-4-chlor-buttersäureethylester

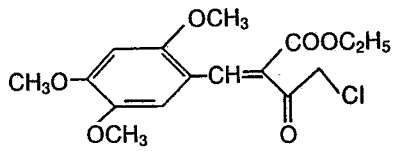

42 g 4-Chloracetessigester, 50 g 2,4,5-Trimethoxybenzaldehyd, 2,5 ml Eisessig und 1 ml Piperidin werden in 60 ml Benzol gelöst und zwei Stunden am Wasserabscheider gekocht. Obwohl der Umsatz noch nicht vollständig ist, wird aufgearbeitet. Man verdünnt mit 200 ml Essigester, extrahiert je dreimal mit gesättigter Natriumbicarbonatlösung, Wasser, 1 m Zitronensäurelösung und nochmals mit Wasser, trocknet und dampft ein. Bei fraktionierter Kristallisation aus Methanol lassen sich zuerst 20 g reines Produkt als E/Z-Gemisch und anschliessend 10,5 g unumgesetzter Aldehyd kristallisieren. Die Mutterlauge enthält neben viel Produkt nur noch wenig Aldehyd und kann nach Vorreinigung durch Chromatographie an Kieselgel ebenfalls kristallisiert werden. Man erhält noch einmal 25 g Produkt.

IR (Nujol): 1715, 1680, 1595, 1505, 1405, 1370, 1340, 1270, 1210, 1165, 1120, 1020 $cm^{-1}$.

NMR ($CDCl_3$): δ = 8,09[1] s, 7,00/6,80[1] s, 6,71[1] s, 4,50/4,41[2] s, 4,32[2] q, J = 7 Hz, 3,98[3] s, 3,88[3] s, 3,83[3] s, 1,36/1,32[3] t, J = 7 Hz ppm.

Beispiel 63
2-(2-Aminothiazol-4-yl)-3-(2,4,5-trimethoxyphenyl)-propensäureethylester (Z-Isomeres)

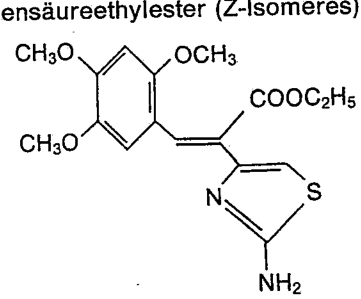

54,8 g des E/Z-Gemisches aus Beispiel 62 werden gemäss Beispiel 25 umgesetzt. Nach Abziehen des Tetrahydrofurans und Einstellen der wässrigen Phase auf pH 8 kristallisiert ein Teil des reinen Z-Isomeren, der aus Aceton umkristallisiert wird, Ausbeute 12,5 g, Fp. 194°. Extraktion der wässrigen Phase mit Essigester, Trocknen der organischen Phase und Eindampfen liefert 44 g E/Z-Gemisch als dunkles Öl.

IR (Nujol): 3320, 1700, 1635, 1600, 1510, 1395, 1320, 1290, 1270, 1230, 1200, 1150, 1110, 1025 $cm^{-1}$.

NMR (DMSO): δ = 7,62[1] s, 7,22[2] s verbreitert, 6,91[1] s, 6,82[1] s, 6,51[1] s, 4,33[2] q, J = 7 Hz, 3,93[6] s, 3,78[3] s, 1,25[3] t, J = 7 Hz ppm.

| | | | | |
|---|---|---|---|---|
| ber.: | C 55,9 | H 5,8 | N 7,7 | S 8,8 |
| gef.: | C 55,7 | H 5,8 | N 7,5 | S 8,8 |

Beispiel 64
2-(2-Aminothiazol-4-yl)-3-(2,4,5-trimethoxyphenyl)propensäureethylesterhydrochlorid (E-Isomeres)

5 g des E/Z-Gemisches aus Beispiel 62 und 1,1 g Thioharnstoff werden in 100 ml Ethanol über Nacht bei Raumtemperatur gerührt und dann 6 Stunden auf 50° erwärmt. Nach dem Abkühlen wird das Produkt abgesaugt, durch Einengen der Mutterlauge und erneutes Absaugen lässt sich eine zweite Fraktion gleicher Reinheit gewinnen. Ausbeute 4,4 g, Fp.: 200° (Zers.).

IR (Nujol): 3220, 1680, 1625, 1595, 1575, 1490, 1400, 1340, 1285, 1255, 1210, 1120, 1040 $cm^{-1}$.

NMR (DMSO): δ = 8,17[1] s, 6,75[2] s, 6,60[1] s, 4,23[2] q, J = 7 Hz, 3,90[6] s, 3,53[3] s, 1,25[3] t, J = 7 Hz ppm.

Beispiel 65
2-(2-Aminothiazol-4-yl)-3-(2,4,5-trimethoxyphenyl)propensäure

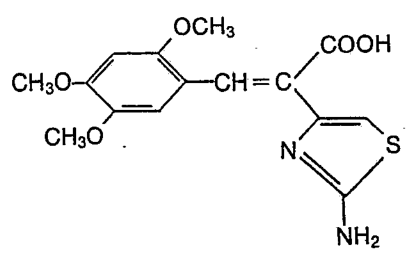

5,5 g des Produkts aus Beispiel 63 werden in 200 ml Dioxan suspendiert und nach Zugabe von 24 ml 2n Natronlauge und 10 ml Wasser 12 Stunden am Rückfluss gekocht. Man zieht das Dioxan ab, extrahiert die wässrige Phase zweimal mit Essigester und stellt auf pH 2,5. Das Produkt wird abgesaugt und getrocknet. Ausbeute 3,4 g eines E/Z-Gemisches.

IR (Nujol): 1650, 1600, 1495, 1330, 1290, 1260, 1206, 1120, 1020 $cm^{-1}$.

E-Isomeres

NMR (DMSO): δ = 7,90[1] s, 6,86[2] s verbreitert, 6,62[1] s, 6,58[1] s, 6,30[1] s, 3,82[3] s, 3,80[3] s, 3,43[3] s ppm.

Z-Isomeres

NMR (DMSO): δ = 7,39[1], 6,98[2] s verbreitert, 6,66[1] s, 6,55[1] s, 6,34[1] s, 3,80[6] s, 3,63[3] s ppm.

Beispiel 66

2-(4-Hydroxybenzyliden)-3-oxo-4-chlorbuttersäureethylester

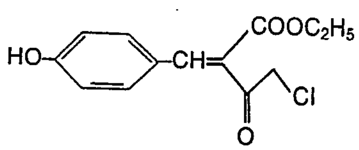

122 g 4-Hydroxybenzaldehyd werden gemäss Beispiel 24 umgesetzt und aufgearbeitet. Nach Destillation von unumgesetztem 4-Chloracetessigester wird das Rohprodukt über Nacht zur Kristallisation stehengelasssen. Man verrührt mit einem Gemisch aus 200 ml Toluol und 50 ml Cyclohexan und saugt von 21 g p-Hydroxybenzaldehyd ab. Die Mutterlauge wird erneut eingedampft und der Rückstand mehrere Tage zur Kristallisation stehengelassen. Nach Behandlung mit Toluol erhält man 85 g eines E/Z-Gemisches, Fp. 92–95°.

IR (Nujol): 3310, 1700, 1665, 1600, 1570, 1505, 1400, 1310, 1285, 1210, 1160, 1010 $cm^{-1}$.

NMR (DMSO): δ = 10,40[1] s breit, 7,76/7,72[1] s, 7,45/7,33[2] d, J = 7 Hz, 6,91/6,88[1] d, J = 7 Hz, 4,96/4,68[2] s, 4,32/4,23[2] q, J = 7 Hz, 1,25[3] t, J = 7 Hz ppm.

| | | | | |
|---|---|---|---|---|
| ber.: | C 58,1 | H 4,9 | Cl 13,2 | O 23,8 |
| gef.: | C 58,2 | H 4,9 | Cl 13,1 | O 24,1 |

Beispiel 67

2-(2-Aminothiazol-4-yl)-3-(4-hydroxyphenyl)propensäureethylesterhydrochlorid (E-Isomeres)

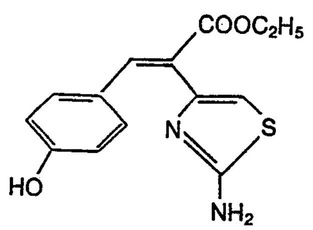

x HCl

83,2 g des Produkts aus Beispiel 66 und 23,6 g Thioharnstoff werden, in 500 ml Ethanol gelöst, 5 Stunden am Rückfluss gekocht. Man dampft ein, arbeitet den Rückstand mit einem Gemisch aus 200 ml Essigester und 30 ml Wasser gut durch und saugt ab. Durch Umlösen des Rückstands aus Methanol erhält man 32 g des reinen E-Isomeren als Hydrochlorid. Fp.: 195–7° (Zers.). Die Essigester/Wasser-Lösung wird mit 2n Natronlauge auf pH 8 gestellt, die Essigesterphase abgetrennt und die wässrige Lösung noch zweimal mit Essigester extrahiert. Trocknen und Eindampfen der organischen Phase liefert ein Öl, aus dem mit Ethanol 8,2 g des E-Isomeren als freie Base kristallisiert werden. Als Mutterlauge verbleibt ein unreines E/Z-Gemisch aus Öl.

IR (Nujol): 1695, 1620, 1590, 1505, 1310, 1270, 1250, 1200, 1160, 1130, 1035 $cm^{-1}$.

NMR (DMSO): δ = 7,93[1] s, 7,27[2] d, J = 8 Hz, 6,93[2] d, J = 8 Hz, 6,86[1] s, 4,26[2] q, J = 7 Hz, 1,26[3] t, J = 7 Hz ppm.

Beispiel 68

2-(2-Aminothiazol-4-yl)-3-(4-hydroxyphenyl)propensäure (Z-Isomeres)

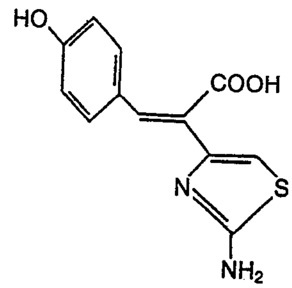

Eine Lösung von 21 g des Produkts aus Beispiel 67 in 300 ml Dioxan wird mit 120 ml 2n Natronlauge versetzt und 5 h auf 50–60° erwärmt und dann 3 Tage bei Raumtemperatur stehengelassen. Aufarbeitung gemäss Beispiel 26 liefert 13 g Produkt. Fp. 178° (Zers.).

IR (Nujol): 1630, 1580, 1510, 1420, 1335, 1305, 1280, 1240, 1170 $cm^{-1}$.

NMR (DMF): δ = 7,39[2] d, J = 8 Hz, 7,07[1] s, 6,78[2] d, J = 8 Hz, 6,45[1] s ppm.

Beispiel 69

2-(5-Methylisoxazol-3-yliden)-3-oxo-4-chlorbuttersäureethylester

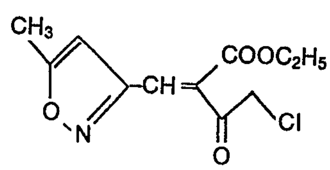

79,5 g 5-Methylisoxazol-3-aldehyd werden gemäss Beispiel 24 mit 4-Chloracetessigester umgesetzt. Man erhält ein Rohprodukt, das gaschromatographisch rein ist. Isomerenverhältnis 2:1.

IR (Film): 2950, 1720, 1590, 1420, 1255, 1225, 1020 $cm^{-1}$.

NMR ($CDCl_3$): δ = 7,64/7,61[1] s, 6,21/6,08[1] s, 4,47[2] s, 4,40/4,32[2] q, J = 7 Hz, 2,43[3] s, 1,32[3] t, J = 7 Hz ppm.

Beispiel 70

2-(2-Aminothiazol-4-yl)-3-(5-methylisoxazol-3-yl)-propensäureethylester

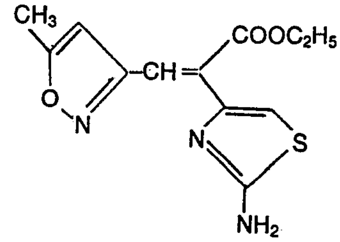

79,5 g des Produkts aus Beispiel 69 werden gemäss Beispiel 25 umgesetzt. Das Rohprodukt wird durch Chromatographie an Kieselgel (Laufmittel Methylenchlorid/Methanol 95:5) gereinigt. Man erhält 74,5 g Isomerengemisch.

Isomeres A

IR (Nujol): 3350, 3250, 3100, 1700, 1625, 1590, 1540, 1250, 1210, 1160, 1040, 1020 $cm^{-1}$.

NMR (DMSO): δ = 7,25[2] s breit, 7,20[1] s, 6,70[1] s, 6,21[1] s, 4,38[2] q, J = 7 Hz, 2,41[3] s, 1,25[3] t, J = 7 Hz ppm.

Beispiel 71

2-(2-Aminothiazol-4-yl)-3-(5-methylisoxazol-3-yl)-propensäure (Z-Isomeres)

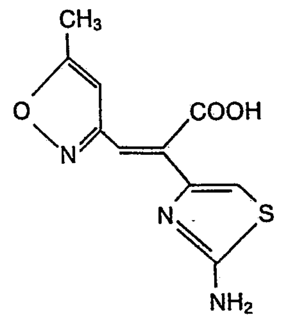

50 g des nach Beispiel 70 hergestellten Isomerengemisches werden in 200 ml Ethanol gelöst. Durch Zugabe von 2n Natronlauge wird auf pH 13,5 eingestellt und 24 h bei pH 13,5 und Raumtemperatur stehengelassen. Man zieht dann das Ethanol ab, gibt 100 ml Wasser zu, stellt auf pH 8 ein und extrahiert dreimal mit Essigester. Eindampfen der organischen Phase liefert 20 g Rohprodukt, die in einem Gemisch aus 70 ml Dioxan und 70 ml 2n Natronlauge gelöst werden. Man kocht 5 Stunden am Rückfluss, arbeitet dann gemäss Beispiel 31 auf und kristallisiert das Rohprodukt aus Aceton/Methanol 1:3 um. Ausbeute 7 g, Fp. 180–2° (Zers.).

IR (Nujol): 1600, 1460, 1400, 1290 $cm^{-1}$.

NMR (DMSO): δ = 7,02[1] s, 6,63[1] s, 6,26[1] s, 2,41[3] s ppm.

Beispiel 72

2-Naphthyliden-3-oxo-4-chlorbuttersäureethylester

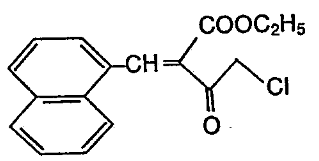

9,85 g 1-Naphthaldehyd werden gemäss Beispiel 24 innerhalb von 10 h mit 15,2 g 4-Chloracetessigester umgesetzt. Nachdem im Hochvakuum andestilliert wurde, verbleibt als Rückstand ein 2:1-Isomerengemisch, das noch Reste Aldehyd enthält.

NMR ($CDCl_3$): δ = 8,12/8,06[1] s, 7,33–8,0[7] m, 7,26/7,20[1] s, 4,55/4,05[2] s, 4,39/5,10[2] q, J = 7 Hz, 1,33/0,90[3] t, J = 7 Hz ppm.

Beispiel 73

2-(2-Aminothiazol-4-yl)-3-naphthylpropensäureethylester

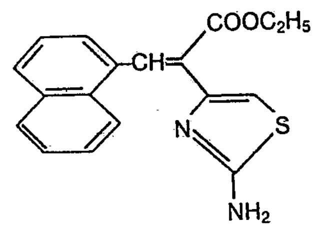

18 g des nach Beispiel 72 hergestellten Rohprodukts werden mit 4,6 g Thioharnstoff in 100 ml Ethanol 3 h bei 50° gerührt. Aufarbeitung gemäss Beispiel 44 liefert ein Rohprodukt, aus dem mit Ether 6,3 g des E-Isomeren kristallisiert werden, Fp. 155–7°. In der Mutterlauge verbleibt ein E/Z-Gemisch.

E-Isomeres

NMR (DMSO): δ = 8,18[1] s, 7,1–8,1[7] m, 6,85[2] s verbreitert, 6,20[1] s, 4,26[2] q, J = 7 Hz, 1,28[3] t, J = 7 Hz ppm.

Z-Isomeres

NMR (DMSO): δ = 8,06[1] s, 7,1–8,1[7] m, 7,26[2] s verbreitert, 6,70[1] s, 4,08[2] q, J = 7 Hz, 0,90[3] t, J = 7 Hz ppm.

Beispiel 74

7-[2-(2-Aminothiazol-4-yl)-2-(2,3,6-trichlorbenzyliden)acetamido]-3-(1-methyl-1-H-tetrazol-5-yl)thiomethyl-3-cefem-4-carbonsäure (Z-Isomeres)

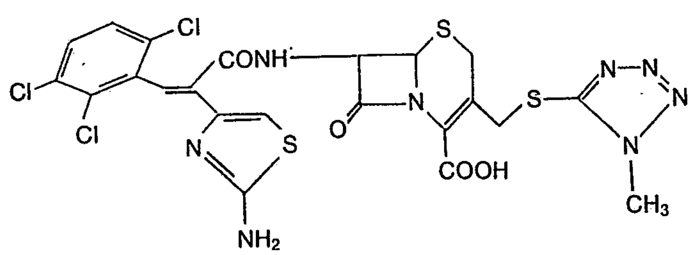

0,7 g 7-Amino-3-(1-methyl-1-H-tetrazol-5-yl)thiomethyl-3-cefem-4-carbonsäure werden in einem Gemisch aus 20 ml THF und 20 ml Wasser durch Zugabe von Triethylamin bei pH 7–8 gelöst. Bei 0° wird 1 g des Produkts aus Beispiel 53 zugegeben und die Lösung dann 3 Stunden bei Raumtemperatur gerührt, wobei der pH-Wert durch Zugabe von Triethylamin auf 7,5 gehalten wird. Nach Abziehen des THF wird pH 3,0 eingestellt und die wässrige Suspension 5 Stunden auf 50° erwärmt.

Man dekantiert von evtl. abgeschiedenem zähen Rückstand, stellt die wässrige Phase auf pH 2,8 und saugt ab. Ausbeute 0,4 g.

IR (KBr): 1785, 1630, 1517, 1436, 1385, 1235, 1178 $cm^{-1}$.

NMR ($DC_3OD$): δ = 7,52[1] d, J = 8 Hz, 7,42[[1] d, J = 8 Hz, 7,16[1] s, 7,10[1] s, 5,69[1] d, J = 5 Hz, 5,03[1] d, J = 5 Hz, 4,31[2] s breit, 4,01[3] s, 3,78[1] d, J = 18 Hz, 3,65[1] d, J = 18 Hz ppm.

Beispiel 75

Natrium-7-[2-(2-aminothiazol-4-yl)-2-(2,6-dichlorbenzyliden)acetamido]-3-(1-methyl-1-H-tetrazol-5-yl)thiomethyl-3-cefem-4-carboxylat (Z-Isomeres)

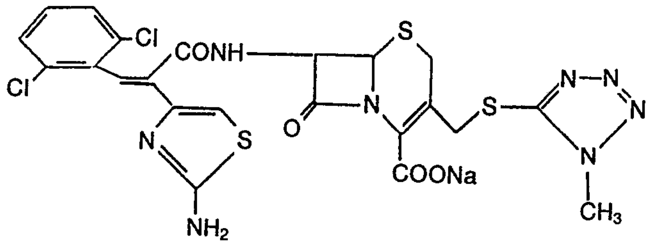

2 g des Produkts aus Beispiel 54 werden analog Beispiel 38 mit 1,8 g 7-Amino-3-(1-methyl-1-H-tetrazol-5-yl)thiomethyl-3-cefem-4-carbonsäure umgesetzt.

Man erhält 1,6 g Natriumsalz.

IR (Nujol): 1750, 1595 $cm^{-1}$.

NMR ($CD_3OD$): δ = 7,34[1] s, 7,27[3] m, 6,73[1] s, 5,62[1] d, J = 5 Hz, 4,95[1] d, J = 5 Hz, 4,39[1] d, J = 13 Hz, 4,29[1] d, J = 13 Hz, 3,97[3] s, 3,66[1] d, J = 18 Hz, 3,42[1] d, J = 18 Hz ppm.

Beispiel 76

2-(2-Aminothiazol-4-yl)-3-naphthylpropensäure (Z-Isomeres)

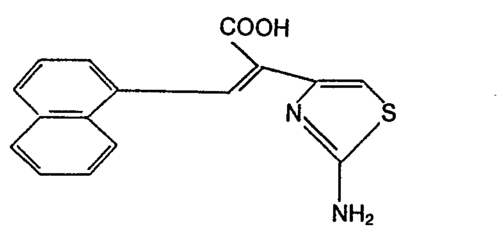

Eine Lösung von 10 g des Isomeren-Gemischs aus Beispiel 73 in 50 ml Ethanol wird mit 2 n Natronlauge auf pH 13,5 gestellt und 10 h bei Raumtemperatur gerührt. Man zieht den Alkohol ab, versetzt mit Wasser und 2 n Salzsäure bis pH 8 und extrahiert dreimal mit Essigester. Die vereinigten Extrakte werden einmal mit Wassser gewaschen und eingedampft. Der Rückstand wird in 100 ml Dioxan aufgenommen, die Lösung mit 30 ml konz. Natronlauge versetzt und 15 Stunden am Rückfluss gekocht. Nach Konzentrieren der Lösung wird mit 15proz. Salzsäure unter Kühlung auf pH 10 eingestellt und die wässrige Lösung dreimal mit Essigester extrahiert. Die wässrige Phase wird anschliessend bis auf pH 2,8 angesäuert und das ausgefallene Produkt abgesaugt. Umkristallisation aus Methanol/Aceton liefert 2,5 g reines Z-Isomeres, Fp. > 220°.

IR (Nujol): 1620, 1595, 1560, 1420, 1300, 1208 $cm^{-1}$.

NMR (DMSO): δ = 7,40–8,18[8] m, 7,17[2] s breit, 6,62[1] s ppm.

Beispiel 77

Natrium-7-[2-(2-aminothiazol-4-yl)-2-naphthylidenacetamido]penam-4-carboxylat (Z-Isomeres)

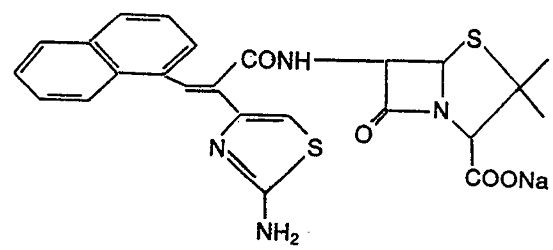

Zu einer Suspension von 0,8 g des Produkts aus Beispiel 76 in 20 ml abs. Methylenchlorid werden bei 0° 24 µl Wasser und anschliessend 1,1 g Phosphorpentachlorid gegeben. Man rührt 2½ Stunden bei Raumtemperatur, saugt ab und wäscht den Rückstand nochmals mit Methylenchlorid. Dieser Rückstand wird zu einer auf 0° gekühlten Lösung von 310 mg 6-Aminopenicillansäure und 600 ml Triethylamin in 60 ml abs. Methylenchlorid gegeben. Man rührt 30 Minuten bei 0° dann 3½ h bei Raumtemperatur nach und arbeitet analog zu Beispiel 38 auf.

Ausbeute 0,8 g Natriumsalz.

IR (Nujol): 1755, 1600 $cm^{-1}$.

NMR ($CD_3OD$): δ = 8,04[1] m, 7,88[1] s, 7,68–7,80[2] m, 7,26–7,54[4] m, 6,53[1] s, 5,34[1] d, J = 5 Hz, 5,28[1] d, J = 5 Hz, 3,91[1] s, 1,32[3] s, 1,22[3] s ppm.

Beispiel 78

Natrium-7-[2-(2-aminothiazol-4-yl)-2-naphthylidenacetamido]-3-acetoxymethyl-3-cefem-4-carboxylat (Z-Isomeres)

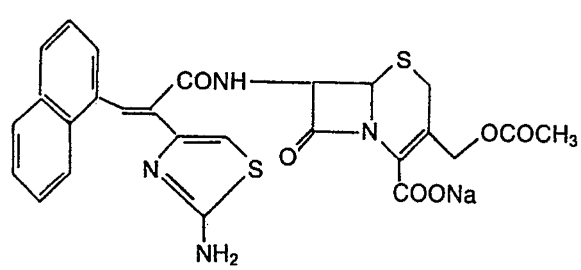

1 g des Produkts aus Beispiel 76 wird analog Beispiel 29 umgesetzt, man erhält 0,8 g Natriumsalz.

IR (Nujol): 1745, 1600 $cm^{-1}$.

NMR ($CD_3OD$): δ = 820[1] m, 8,06[1] s, 7,38–7,95[6] m, 6,66[1] s, 5,74[1] d, J = 5 Hz, 4,99[1] d, J = 5 Hz, 4,97[1] d, J = 13 Hz, 4,78[1] d, J = 13 Hz, 3,46[1] d, J = 17 Hz, 3,11[1] d, J = 17 Hz, 2,01[3] s ppm.

Beispiel 79

Natrium-7-[2-(2-aminothiazol-4-yl)-2-(5-methylisoxazol-3-yliden)acetamido]-3-acetoxymethyl-3-cefem-4-carboxylat (Z-Isomeres)

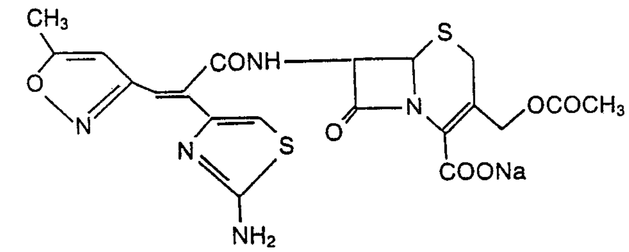

1 g des Produkts aus Beispiel 71 wird analog Beispiel 29 umgesetzt. Ausbeute 1,5 g Natriumsalz.

IR (Nujol): 1755, 1600, 1520, 1230 $cm^{-1}$.

NMR ($CD_3OD$): δ = 7,21[1] s, 6,70[1] s, 6,30[1] s, 5,91[1] d, J = 5 Hz, 5,16[1] d, J = 5 Hz, 5,00[1] d, J = 12 Hz, 4,81[1] d, J = 12 Hz, 3,61[1] d, J = 18 Hz, 3,34[1] d, J = 18 Hz, 2,41[3] s, 2,03[3] s ppm.

Beispiel 80

7-[2-(2-Aminothiazol-4-yl)-2-(4-hydroxybenzyliden)acetamido]-3-acetoxymethyl-3-cefem-4-carbonsäure

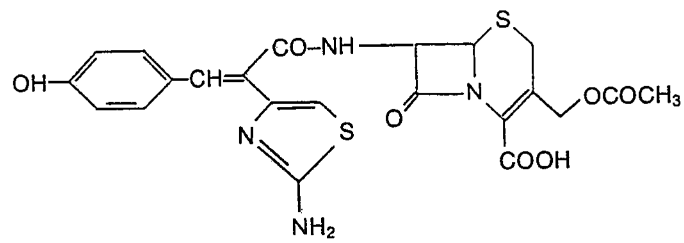

3 g des Produkts aus Beispiel 68 werden analog Beispiel 29 umgesetzt. Man erhält 4,5 g eines E/Z-Gemischs. 1 g dieses Gemischs wird in 25 ml Methylenchlorid suspendiert, mit 500 µl Triethylamin versetzt und über Nacht bei Raumtemperatur gerührt. Man saugt ab, suspendiert den Rückstand in 20 ml Wasser und stellt den pH mit 2 n Salzsäure auf 3,0 ein. Das Produkt wird abgesaugt und getrocknet. Ausbeute 0,6 g.

IR (Nujol): 1765, 1720, 1650, 1590, 1500, 1225, 1165 $cm^{-1}$.

NMR (DMSO): δ = 8,56/8,15[1] d, J = 8 Hz, 7,0–7,8[5] m, 6,72[1] t, J = 7 Hz, 6,40/6,29[1] s, 5,84[1] m, 5,21/5,15[1] d, J = 5 Hz, 5,02[1] d, J = 12 Hz, 4,68[1] d, J = 12 Hz, 3,66[1] d, J = 17 Hz, 3,49[1] d, J = 17 Hz, 2,05[3] s ppm.

Beispiel 81

2-Ethyliden-3-oxo-4-chlorbuttersäureethylester

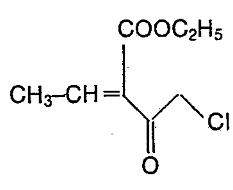

1 g Piperidin wurde in eine gerührte Mischung von 82 g 4-Chloracetoessigsäure und 24 g Acetaldehyd bei −20 °C getropft. Nachdem die Mischung bei −10 bis −20 °C für 7 Stunden gerührt worden war, wurden 100 ml Ethylacetat hinzugefügt. Danach wurde die Lösung dreimal mit eiskalter 1 normaler Salzsäure und mit Wasser extrahiert. Die organische Phase wurde über Magnesiumsulfat getrocknet und konzentriert. Das zurückbleibende Öl wurde einer Kugelrohrdestillation unter Hochvakuum unterworfen. Ausbeute 37 g. $Kp_{0,05}$: 110 °C

Das erhaltene Produkt war eine Z/E-Mischung.

IR ($CHCl_3$): 1700, 1620, 1445, 1375, 1260 $cm^{-1}$.

NMR ($CDCl_3$): δ = 7,30/7,20[1] q, J = 8 Hz, 4,45/4,43[2] s, 4,36/4,30[2] q, J = 8 Hz, 2,10/1,96[3] d, J = 8 Hz, 1,35/1,30[3] t, J = 7 Hz ppm.

Beispiel 82

1-(2-Aminothiazol-4-yl)-1-propencarbonsäureethylester

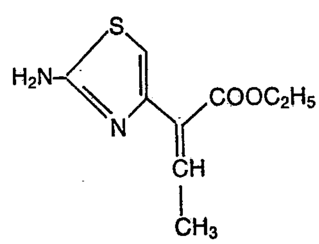

Zu einer Lösung von 190 g des Produkts aus Beispiel 81 in 400 ml Tetrahydrofuran werden

nacheinander eine Lösung von 82 g Natriumacetat in 150 ml Wasser und dann 76 g Thioharnstoff gegeben. Man rührt bei Raumtemperatur 20 Stunden, zieht dann das THF ab und versetzt den Rückstand mit Essigester.

Nach Abtrennen des Wassers wird die organische Phase noch zweimal mit Wasser, einmal mit 1 m Zitronensäurelösung und einmal mit Wasser extrahiert, getrocknet und eingedampft. Das Produkt wird durch Chromatographie am Kieselgel (Laufmittel Methylenchlorid) gereinigt. Ausbeute 10,3 g.

Beispiel 83
1-(2-Aminothiazol-4-yl)-1-propencarbonsäure

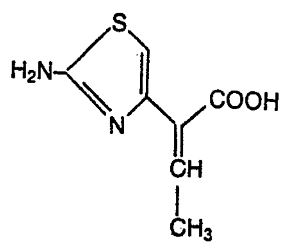

Das Produkt aus Beispiel 82 wird in einem Gemisch aus 20 ml Methanol und 5 ml 2 n Natronlauge gelöst.

Man kocht die Lösung 6 Stunden am Rückfluss und arbeitet dann gemäss Beispiel 15 auf. Ausbeute 2,2 g.

E-Isomeres:
NMR (DMSO): δ = 7,04[2] s verbreitert, 6,89[1] q, J = 7 Hz, 6,48[1] s, 1,88[6] d, J = 7 Hz ppm.

Z-Isomeres:
NMR (DMSO): δ = 6,54[1] q, J = 7 Hz, 6,48[1] s, 1,81[6] d, J = 7 Hz ppm.

Beispiel 84
7-[1-(2-Aminothiazol-4-yl)-1-propencarbamido]-3-acetoxymethyl-3-cefem-4-carbonsäure

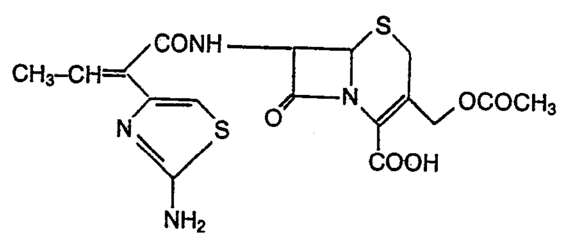

Z-Isomeres
1 g des Z-Isomeren aus Beispiel 83 wird analog Beispiel 19 umgesetzt, man erhält 0,6 g Produkt.
NMR (DMSO): δ = 9,39[1] d, J = 7 Hz, 7,10[2] s breit, 6,33[1] q, J = 7 Hz, 6,24[1] s, 5,82[1] dd, J = 4 Hz, J = 7 Hz, 5,20[1] d, J = 4 Hz, 5,01[1] d, J = 13 Hz, 4,71[1] d, J = 13 Hz, 5,66[1] d, J = 18 Hz, 3,52[1] d, J = 18 Hz, 2,06[3] s, 1,84[6] d, J = 7 Hz ppm.

E-Isomeres
1 g des E-Isomeren aus Beispiel 83 wird analog Beispiel 19 umgesetzt, man erhält 0,6 g Produkt.
NMR (DMSO): δ = 9,20[1] d, J = 9 Hz, 7,90[2] s, sehr breit, 6,87[1] q, J = 7 Hz, 6,64[1] s, 5,81[1] dd, J = 9 Hz, J = 5 Hz, 5,15[1] d, J = 5 Hz, 5,02[1] d, J = 13 Hz, 4,67[1] d, J = 13 Hz, 3,65[1] d, J = 18 Hz, 3,50[1] d, J = 18 Hz, 2,03[3] s, 1,90[6] d, J = 7 Hz ppm.

Auf dem gleichen Wege wie das Produkt aus Beispiel 84 wurde hergestellt:
7-[1-(2-Aminothiazol-4-yl)-1-butencarbamido]-3-acetoxymethyl-3-cefem-4-carbonsäure

Z-Isomeres
NMR (DMSO): δ = 9,25[1] d, J = 7 Hz, 7,00[2] s breit, 6,23[1] t, J = 7 Hz, 6,19[1] s, 5,79[1] dd, J = 7 Hz, J = 6 Hz, 5,17[[1] d, J = 6 Hz, 4,98[1] d, J = 12 Hz, 4,67[1] d, J = 12 Hz, 3,62[1] d, J = 18 Hz, 3,50[1] d, J = 18 Hz, 2,18[2] dq, J = 7 Hz, J = 7 Hz, 2,04[3] s, 1,01[3] t, J = 7 Hz ppm.

E-Isomeres
NMR (DMSO): δ = 9,08[1] d, J = 8 Hz, 8,0[2] s sehr breit, 6,77[1] t, J = 7 Hz, 6,63[1] s, 5,80[1] dd, J = 8 Hz, J = 5 Hz, 5,15[1] d, J = 5 Hz, 5,01[1] d, J = 13 Hz, 4,69[1] d, J = 13 Hz, 3,66[1] d, J = 18 Hz, 3,49[1] d, J = 18 Hz, 2,29[2] dq, J = 7 Hz, J = 7 Hz, 2,05[3] s, 1,02[3] t, J = 7 Hz ppm.

Analog wurden hergestellt:
7-[1-(2-Aminothiazol-4-yl)-1-undecencarbamido]-3-acetoxymethyl-3-cefem-4-carbonsäure
7-[1-(2-Aminothiazol-4-yl)-3,3-dimethyl-1-butencarbamido]-3-acetoxymethyl-3-cefem-4-carbonsäure
7-[1-(2-Aminothiazol-4-yl)-3methyl-1-butencarbamido]-3-acetoxymethyl-3-cefem-4-carbonsäure
7-[1-(2-Aminothiazol-4-yl)-2-cyclohexyl-1-ethencarbamido]-3-acetoxymethyl-3-cefem-4-carbonsäure
7-[1-(2-Aminothiazol-4-yl)-2-cyclopentyl-1-ethencarbamido]-3-acetoxymethyl-3-cefem-4-carbonsäure
7-[1-(2-Aminothiazol-4-yl)-2-cycloheptyl-1-carbamido]-3-acetoxymethyl-3-cefem-4-carbonsäure
7-[1-(2-Aminothiazol-4-yl)-3-methoxy-1-propencarbamido]-3-acetoxymethyl-3-cefem-4-carbonsäure
7-[1-(2-Aminothiazol-4-yl)-3-ethoxy-1-propencarbamido]-3-acetoxymethyl-3-cefem-4-carbonsäure
7-[1-(2-Aminothiazol-4-yl)-4-dimethylamino-3,3-dimethyl-1-butencarbamido]-3-acetoxymethyl-3-cefem-4-carbonsäure
7-[1-(2-Aminothiazol-4-yl)-3-dimethylaminomethyl-3-methyl-1-hexencarbamido]-3-acetoxymethyl-3-cefem-4-carbonsäure
6-[1-(2-Aminothiazol-4-yl)-3,3-dimethyl-1-butencarbamido]-penamcarbonsäure
7-[1-(2-Aminothiazol-4-yl)-3,3-dimethyl-1-butencarbamido]-3-(1-methyl-1-H-tetrazol-5-yl)thiomethyl-3-cefem-4-carbonsäure
6-[1-(2-Aminothiazol-4-yl)-1-propencarbamido]-penamcarbonsäure
7-[1-(2-Aminothiazol-4-yl)-1-propencarbamido]-3-(1-methyl-1-H-tetrazol-5-yl)thiomethyl-3-cefem-4-carbonsäure

7-[1-(2-Aminothiazol-4-yl)-4-dimethylamino-3,3-dimethyl-1-butencarbamido]-3-(1-methyl-1-H-tetrazol-5-yl)thiomethyl-3-cefem-4-carbonsäure

6-[1-(2-Aminothiazol-4-yl)-4-dimethylamino-3,3-dimethyl-1-butencarbamido]penamcarbonsäure

6-[1-(2-Aminothiazol-4-yl)-3-methoxy-1-propencarbamido]penamcarbonsäure

7-[1-(2-Aminothiazol-4-yl)-3-methoxy-1-propencarbamido]-3-(1-methyl-1-H-tetrazol-5-yl)-thiomethyl-3-cefem-4-carbonsäure

7-[1-(2-Aminothiazol-4-yl)-1-pentencarbamido]-3-acetoxymethyl-3-cefem-4-carbonsäure

7-[1-(2-Aminothiazol-4-yl)-1-hexencarbamido]-3-acetoxymethyl-3-cefem-4-carbonsäure

7-[1-(2-Aminothiazol-4-yl)-1-heptencarbamido]-3-acetoxymethyl-3-cefem-4-carbonsäure

7-[1-(2-Aminothiazol-4-yl)-1-octencarbamido]-3-acetoxymethyl-3-cefem-4-carbonsäure

7-[1-(2-Aminothiazol-4-yl)-1-nonencarbamido]-3-acetoxymethyl-3-cefem-4-carbonsäure

7-[1-(2-Aminothiazol-4-yl)-1-decencarbamido]-3-acetoxymethyl-3-cefem-4-carbonsäure

**Patentansprüche**

1. Verbindungen der Formel (I)

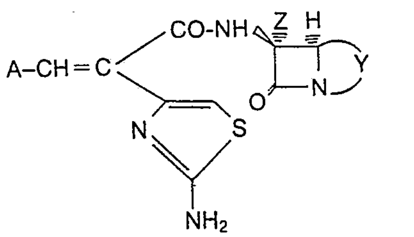 (I)

worin

A Wasserstoff, gegebenenfalls cyclisches, Alkyl, Alkenyl oder Alkinyl mit bis zu 18 C-Atomen, die durch O, S oder Methoxyimino substituiert sein können, einen Rest der Formel

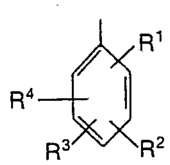

bedeutet,

worin $R^1$, $R^2$, $R^3$ und $R^4$ unabhängig voneinander Wasserstoff, Halogen, Alkyl mit bis zu 6 Kohlenstoffatomen, Aryl, eine Gruppe $-OCOR^5$, eine Gruppe 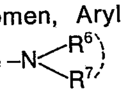 ,

Nitro, Cyano, Trifluormethyl, Hydroxy, Alkoxy oder Alkylthio mit bis zu 6 C-Atomen, Hydroxycarbonyl, Alkoxycarbonyl mit bis zu 6 C-Atomen im Alkylteil, Aminocarbonyloxy, Sulfonyl oder Sulfo bedeuten,

worin wiederum Aryl einen substituierten oder unsubstituierten carbocyclischen aromatischen Ring oder 5- oder 6-gliedrigen heterocyclischen Ring bedeutet,

und worin $R^5$, gegebenenfalls substituiertes und gegebenenfalls cyclisches, Alkyl, Alkenyl oder Alkinyl mit bis zu 6 C-Atomen bedeutet,

und worin $R^6$ und $R^7$ unabhängig voneinander Wasserstoff, gegebenenfalls substituiertes und gegebenenfalls cyclisches, Alkyl, Alkenyl oder Alkinyl mit bis zu 6 C-Atomen oder Alkanoyl mit bis zu 6 C-Atomen oder gemeinsam Alkyl oder Alkanoyl mit bis zu 6 C-Atomen bedeuten, oder Naphthyl, 5-Methylisoxazol-3-yl, Furyl, Thienyl oder Pyridinyl bedeutet,

und worin

Y in der Form der freien Säure einen Rest der Formel

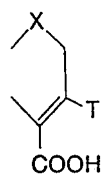 oder 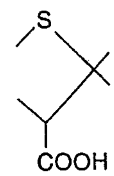

darstellt,

worin X Schwefel oder Sauerstoff bedeutet und T ein in der Cephalosporinchemie üblicher Rest ist,

und worin

Z H oder Methoxy bedeutet.

2. Verbindungen nach Anspruch 1, dadurch gekennzeichnet, dass

T Wasserstoff, $C_1$–$C_4$-Alkyl, Halogen, $C_1$–$C_4$-Alkoxy, Hydroxymethyl, Formyloxymethyl, $C_1$–$C_4$-Alkylcarbonyloxymethyl, Aminocarbonyloxymethyl, Pyridiniummethyl, 4-Carbamoylpyridiniummethyl oder Heterocyclylthiomethyl bedeutet, wobei Heterocyclyl für einen Rest der Formel

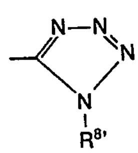 , 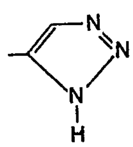 , 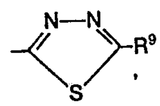 , 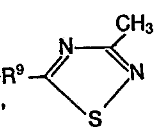

steht, worin

$R^8$ Wasserstoff, Methyl, 2-Dimethylaminoethyl, Carboxymethyl oder Sulfomethyl und

$R^9$ Wasserstoff oder Methyl bedeuten.

3. Verbindungen nach den Ansprüchen 1 und 2, dadurch gekennzeichnet, dass

Z Wasserstoff oder Methoxy,

X Schwefel,

T einen Rest $-CH_2OCOCH_3$,

$-CH_2OCOCH_3$, $-CH_2-$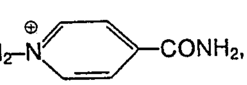, $-CH_2-S-$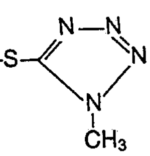 ,

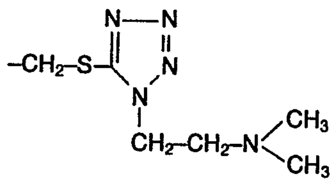 oder $-CH_2OCONH_2$

und

A einen Rest der Formel

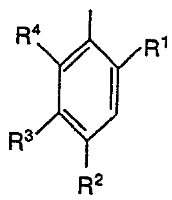 , 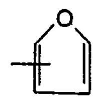 , 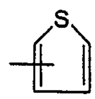 , 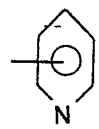 , 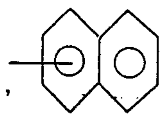

wobei $R^1$ bis $R^4$ die in Anspruch 1 genannte Bedeutung haben, bedeutet.

4. Pharmazeutisch verträgliche Salze der Verbindungen gemäss den Ansprüchen 1 bis 3.

5. Verbindungen nach einem der Ansprüche 1 bis 4 in der Z-Konfiguration.

6. Verfahren zur Herstellung der Verbindungen der Formel (I)

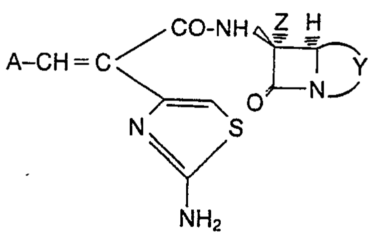 (I)

worin

A Wasserstoff, gegebenenfalls cyclisches, Alkyl, Alkenyl oder Alkinyl mit bis zu 18 C-Atomen, die durch O, S oder Methoxyimino substituiert sein können, einen Rest der Formel

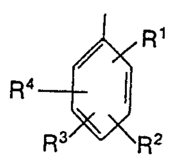 ,

bedeutet,

worin $R^1$, $R^2$, $R^3$ und $R^4$ unabhängig voneinander Wasserstoff, Halogen, Alkyl mit bis zu 6 Kohlenstoffatomen, Aryl, eine Gruppe $-OCOR^5$, eine Gruppe 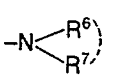 ,

Nitro, Cyano, Trifluormethyl, Hydroxy, Alkoxy oder Alkylthio mit bis zu 6 C-Atomen, Hydroxycarbonyl, Alkoxycarbonyl mit bis zu 6 C-Atomen im Alkylteil, Aminocarbonyloxy, Sulfonyl oder Sulfo bedeuten,

worin wiederum Aryl einen substituierten oder unsubstituierten carbocyclischen aromatischen Ring oder 5- oder 6-gliedrigen heterocyclischen Ring bedeutet,

und worin $R^5$, gegebenenfalls substituiertes und gegebenenfalls cyclisches, Alkyl, Alkenyl oder Alkinyl mit bis zu 6 C-Atomen bedeutet,

und worin $R^6$ und $R^7$ unabhängig voneinander Wasserstoff, gegebenenfalls substituiertes und gegebenenfalls, cyclisches Alkyl, Alkenyl oder Alkinyl mit bis zu 6 C-Atomen oder Alkanoyl mit bis zu 6 C-Atomen oder gemeinsam Alkyl oder Alkanoyl mit bis zu 6 C-Atomen bedeuten, oder Naphthyl, 5-Methylisoxazol-3-yl, Furyl, Thienyl oder Pyridinyl bedeutet,

und worin

Y in der Form der freien Säure einen Rest der Formel

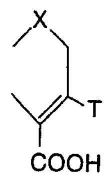 oder 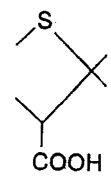

darstellt,

worin X Schwefel oder Sauerstoff bedeutet und T ein in der Cephalosporinchemie üblicher Rest ist,

und worin

Z H oder Methoxy bedeutet,

dadurch gekennzeichnet, dass Verbindungen der allgemeinen Formel (II)

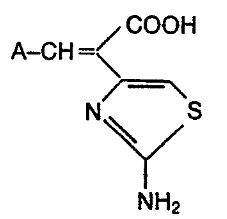 (II)

worin A die oben angegebene Bedeutung hat, in denen die Aminogruppe geschützt oder ungeschützt vorliegen kann, nach Aktivierung der Carboxylgruppe durch Überführung in ein gemischtes Anhydrid oder nach Überführung in das Säurehalogenid oder nach Überführung in einen aktivierten Ester mit Verbindungen der allgemeinen Formel (III), gegebenenfalls in der Mono- oder Disilylform oder in Form von spaltbaren Estern

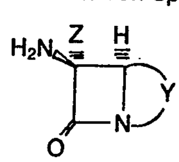 III,

worin Y und Z die obengenannte Bedeutung besitzen, zur Umsetzung gebracht werden und anschliessend Schutzgruppen gegebenenfalls entfernt werden.

7. Verfahren nach Anspruch 6, dadurch gekennzeichnet, dass Verbindungen der Formel (II) nach Aktivierung der Carboxylgruppe mit Phosphorpentachlorid oder Phosphoroxychlorid oder als Ester mit einem Alkohol der Formel (IV)

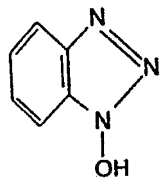

IV,

ohne vorherige Einführung einer Amino-Schutzgruppe, mit einer Verbindung der Formel (III) umgesetzt werden.

8. Verfahren zur Herstellung von Verbindungen der Formel (I), dadurch gekennzeichnet, dass man Verbindungen der Formel (I) gemäss Anspruch 6, in denen T die Bedeutung von $-CH_2-OCO-C_1-C_4$-Alkyl hat, mit Nukleophilen, insbesondere

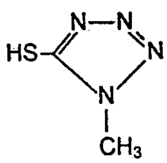

oder

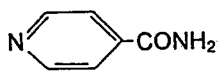

umsetzt.

9. Verfahren zur Herstellung von Verbindungen der Formel (I), dadurch gekennzeichnet, dass man Verbindungen der Formel (I) gemäss Anspruch 6, in denen T die Bedeutung von $-CH_2OH$ hat, mit $O=C=NSO_2Cl$ oder $O=C=N-COCCl_3$ umsetzt und anschliessend die Gruppe $-SO_2Cl$ oder $-COCCl_3$ abspaltet.

10. Verbindungen der allgemeinen Formel

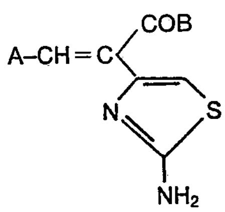

in der A die in Anspruch 1 angegebene Bedeutung hat und B OH, Halogen, eine Carboxylgruppen aktivierende Gruppierung oder eine $C_1-C_4$-Alkoxygruppe darstellt.

11. Verfahren zur Herstellung von Verbindungen der Formel (VIII)

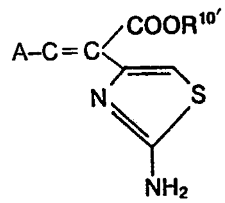

VIII,

worin

$R^{10'}$ $C_1-C_4$-Alkyl bzw. H bedeutet und

A die in Anspruch 6 angegebene Bedeutung hat, dadurch gekennzeichnet, dass man in einem ersten Schritt eine Verbindung der Formel (V)

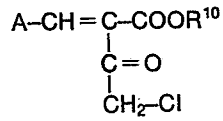

V,

in der A die obengenannte Bedeutung hat und $R^{10}$ $C_1-C_4$-Niederalkyl, insbesondere Ethyl, bedeutet, durch Kondensation einer Verbindung der Formel (VI)

$$Cl-CH_2-CO-CH_2-COOR^{10}, \quad (VI)$$

in der $R^{10}$ die obengenannte Bedeutung hat, mit einer Carbonylverbindung der Formel (VII)

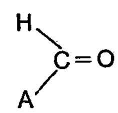

VII,

in der A die obengenannte Bedeutung hat, herstellt und die Verbindung der Formel (V) anschliessend mit Thioharnstoff umsetzt und eine Verbindung der Formel (VIII) gegebenenfalls zur freien Carbonsäure verseift.

12. Verfahren zur Herstellung der Verbindungen gemäss Formel (VIII) nach Anspruch 11, worin $R^{10'}$ Ethyl bedeutet, dadurch gekennzeichnet, dass man das Kondensationsprodukt IX aus Acetessigester und einer Carbonylverbindung der Formel (VII) mit Brom zu einem 1,4-Dibromid X nach folgendem Schema reagieren lässt:

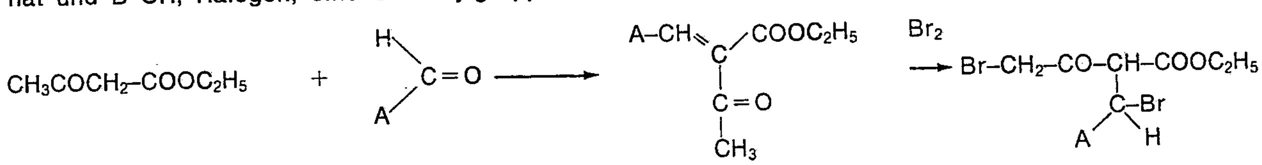


VII IX X,

und die Verbindung der Formel (X) mit Thioharnstoff unter gleichzeitiger Cyclisierung und HBr-Eliminierung umsetzt.

13. Verfahren zur Herstellung der Verbindungen gemäss Formel (VIII) nach Anspruch 11, worin $R^{10'}$ Wasserstoff bedeutet und die Aminogruppe des Aminothiazolringes geschützt ist, dadurch gekennzeichnet, dass man die Verbindungen der allgemeinen Formel (XII)

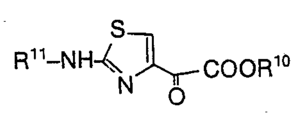

XII

in der $R^{10}$ die in Anspruch 11 genannte Bedeutung hat und $R^{11}$ eine Amino-Schutzgruppe wie Formyl oder tert.-Butoxycarbonyl darstellt, mit einem Phosphonat der allgemeinen Formel (XIII)

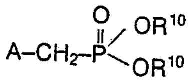

XIII

in der A und $R^{10}$ die in Anspruch 11 genannten Bedeutungen haben,
nach Deprotonierung von (XIII) mit einer Base wie Natriumhydrid, Lithiumdiisopropylamid, Lithiumhydrid, Butyllithium oder Kalium-tert.-butanolat, in einem organischen Lösungsmittel wie Tetrahydrofuran zu einer Verbindung der allgemeinen Formel (XIV)

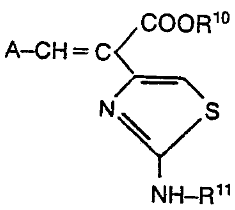

XIV

umsetzt und aus dieser Verbindung (XIV) durch alkalische Verseifung eine Carbonsäure der Formel (VIII) gewinnt, worin $R^{10'}$ Wasserstoff bedeutet und die Aminogruppe des Aminothiazolringes geschützt ist.

14. Verwendung von Verbindungen der Formel (I) gemäss Anspruch 1 zur Herstellung von Arzneimitteln.

**Claims**

1. Compounds of the formula (I)

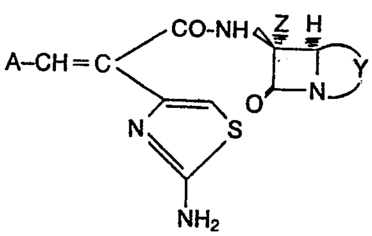

(I)

wherein
A denotes hydrogen, optionally cyclic, alkyl, alkenyl or alkinyl with up to 18 C atoms, any of which may be substituted by O, S or methoxyimino, or denotes a radical of the formula

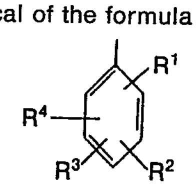

,

wherein
$R^1$, $R^2$, $R^3$ and $R^4$ independently of one another denote hydrogen, halogen, alkyl with up to 6 carbon atoms, aryl, an $-OCOR^5$ group, a 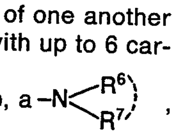 , group, nitro, cyano, trifluoromethyl, hydroxyl, alkoxy or alkylthio with up to 6 C atoms, hydroxycarbonyl, alkoxycarbonyl with up to 6 C atoms in the alkyl part, aminocarbonyloxy, sulphonyl or sulpho,
wherein
aryl in turn denotes a substituted or unsubstituted carbocyclic aromatic ring or 5-membered or 6-membered heterocyclic ring,
and wherein
$R^5$ denotes optionally substituted and optionally cyclic alkyl, alkenyl or alkinyl with up to 6 C atoms,
and wherein
$R^6$ and $R^7$ independently of one another denote hydrogen, optionally substituted and optionally cyclic alkyl, alkenyl or alkinyl with up to 6 C atoms or alkanoyl with up to 6 C atoms, or together denote alkyl or alkanoyl with up to 6 C atoms, or denotes naphthyl, 5-methylisoxazol-3-yl, furyl, thienyl or pyridinyl,
and wherein
Y in the form of the free acid denotes a radical of the formula

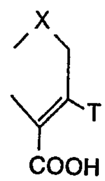

or

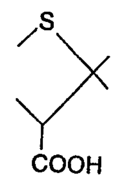

wherein
X denotes sulphur or oxygen and
T is a radical which is conventional in cephalosporin chemistry
and wherein
Z denotes H or methoxy.

2. Compounds according to Claim 1, characterized in that
T denotes hydrogen, $C_1$–$C_4$-alkyl, halogen, $C_1$–$C_4$-alkoxy, hydroxymethyl, formyloxymethyl, $C_1$–$C_4$-alkylcarbonyloxymethyl, aminocarbonyloxymethyl, pyridinium methyl, 4-carbamoylpyridiniummethyl or heterocyclylthiomethyl,
wherein
heterocyclyl represents a radical of the formula

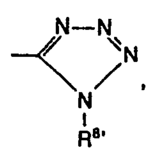 , 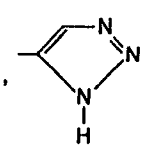 , 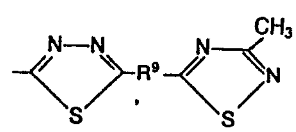

wherein
$R^8$ denotes hydrogen, methyl, 2-dimethylaminoethyl, carboxymethyl or sulphomethyl and
$R^9$ denotes hydrogen or methyl.

3. Compounds according to Claims 1 and 2, characterized in that
Z denotes hydrogen or methoxy,
X denotes sulphur,
T denotes a radical

$-CH_2OCOCH_3$, 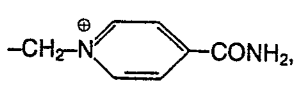, 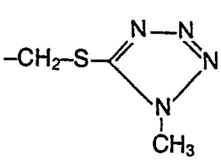 ,

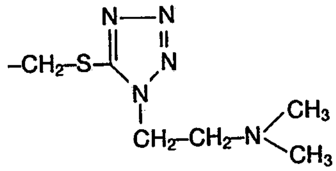 or $-CH_2OCONH_2$

and

A denotes a radical of the formula

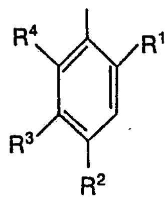 , 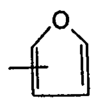 , 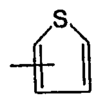 , 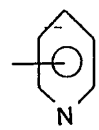 , 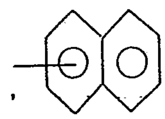

wherein

$R^1$ to $R^4$ have the meaning given in Claim 1.

4. Pharmaceutically acceptable salts of the compounds according to Claims 1 to 3.

5. Compounds according to one of Claims 1 to 4 in the Z-configuration.

6. Process for the preparation of the compounds of the formula (I),

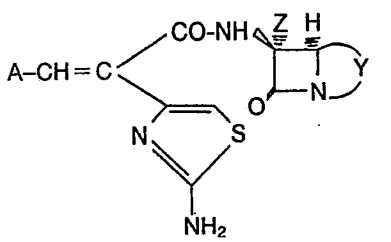 (I)

wherein

A denotes hydrogen, optionally cyclic, alkyl, alkenyl or alkinyl with up to 18 C atoms, any of which may be substituted by O, S or methoxyimino, or denotes a radical of the formula

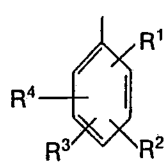 ,

wherein

$R^1$, $R^2$, $R^3$ and $R^4$ independently of one another denote hydrogen, halogen, alkyl with up to 6 carbon atoms, aryl, an $-OCOR^5$ group, a 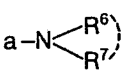 group, nitro, cyano, trifluoromethyl, hydroxyl, alkoxy or alkylthio with up to 6 C atoms, hydroxycarbonyl, alkoxycarbonyl with up to 6 C atoms in the alkyl part, aminocarbonyloxy, sulphonyl or sulpho, wherein

aryl in turn denotes a substituted or unsubstituted carbocyclic aromatic ring or 5-membered or 6-membered heterocyclic ring,

and wherein

$R^5$ denotes optionally substituted and optionally cyclic alkyl, alkenyl or alkinyl with up to 6 C atoms,

and wherein

$R^6$ and $R^7$ independently of one another denote hydrogen, optionally substituted and optionally cyclic alkyl, alkenyl or alkinyl with up to 6 C atoms or alkanoyl with up to 6 C atoms, or together denote alkyl or alkanoyl with up to 6 C atoms, or denotes naphthyl, 5-methylisoxazol-3-yl, furyl, thienyl or pyridinyl,

and wherein

Y in the form of the free acid denotes a radical of the formula

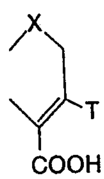 or 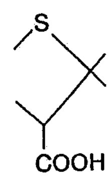

wherein

X denotes sulphur or oxygen and

T is a radical which is conventional in cephalosporin chemistry

and wherein

Z denotes H or methoxy,

characterized in that compounds of the general formula (II)

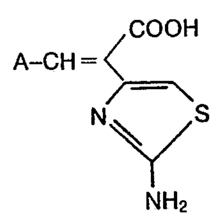 II

wherein

A has the abovementioned meaning,

and in which the amino group can be in the protected or unprotected form, are reacted, after the carboxyl group has been activated by being converted into a mixed anhydride, or after being converted into the acid halide, or after being converted into an activated ester with compounds of the general formula (III), if appropriate in the monosilyl or disilyl form or in the form of esters which can be split

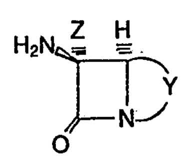 III

wherein

Y and Z have the abovementioned meaning,

and, if appropriate, the protective groups are then removed.

7. Process according to Claim 6, characterized in that compounds of the formula (II) are reacted, after the carboxyl group has been activated with phosphorus pentachloride or phosphorus oxychloride or in the form of esters with an alcohol of the formula (IV)

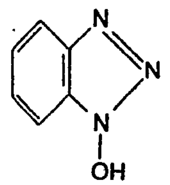

IV,

with a compound of the formula (III) without prior introduction of an amino-protective group.

8. Process for the preparation of compounds of the formula (I), characterized in that compounds of the formula (I) according to Claim 6, in which T denotes $-CH_2-OCO-C_1-C_4$ alkyl are reacted with nucleophiles, in particular

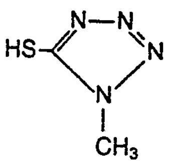

or

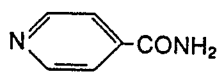

9. Process for the preparation of compounds of the formula (I), characterized in that compounds of the formula (I) according to Claim 6, in which T denotes $-CH_2OH$ are reacted with $O=C=NSO_2Cl$ or $O=C=N-COCCl_3$ and the $-SO_2Cl$ or $-COCCl_3$ group is then split off.

10. Compounds of the general formula

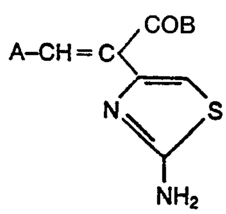

in which

A has the meaning given in Claim 1 and

B represents OH, halogen, a grouping activating carboxyl groups or a $C_1-C_4$-alkoxy group.

11. Process for the preparation of compounds of the formula (VIII)

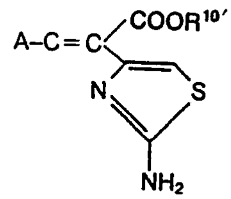

VIII,

wherein

$R^{10'}$ denotes $C_1-C_4$-alkyl or H and

A has the meaning given in Claim 6, characterized in that a compound of the formula (V)

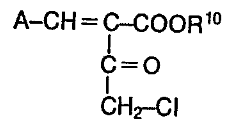

V,

in which

A has the abovementioned meaning and

$R^{10}$ denotes $C_1-C_4$-lower alkyl, in particular ethyl, is prepared, in a first step, by condensation of a compound of the formula (VI)

$$Cl-CH_2-CO-CH_2-COOR^{10} \quad (VI)$$

in which

$R^{10}$ has the abovementioned meaning, with a carbonyl compound of the formula (VII)

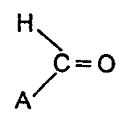

VII,

in which

A has the abovementioned meaning, and the compound of the formula (V) is then reacted with thiourea and a compound of the formula (VIII) is optionally hydrolysed to give the free carboxylic acid.

12. Process for the preparation of compounds according to formula (VIII) according to Claim 11, wherein

$R^{10'}$ denotes ethyl,

characterized in that the condensation product IX of ethyl acetoacetate and a carbonyl compound of the formula (VII) is reacted with bromine to give a 1,4-dibromide X in accordance with the following equation:

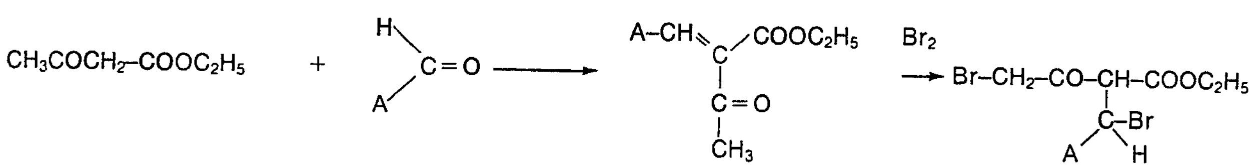


VII IX X,

and the compound of the formula (X) is reacted with thiourea, with simultaneous cyclization and elimination of HBr.

13. Process for the preparation of compounds according to formula (VIII) according to Claim 11, wherein

$R^{10'}$ denotes hydrogen and the amino group of the aminothiazole ring is protected, characterized in that the compounds of the general formula (XII)

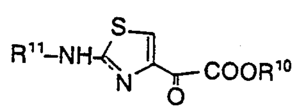

XII

in which

$R^{10}$ has the meaning given in Claim 11 and

$R^{11}$ represents an amino-protective group, such as formyl or tert.-butoxycarbonyl,

is reacted with a phosphonate of the general formula (XIII)

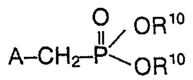

XIII

in which

A and $R^{10}$ have the meanings given in Claim 11, after deprotonation of (XIII) with a base, such as sodium hydride, lithium diisopropylamide, lithium hydride, butyl-lithium or potassium tert.-butanolate, in an organic solvent, such as tetrahydrofuran, to give a compound of the general formula (XIV)

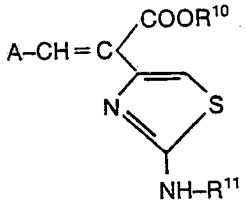

XIV

and a carboxylic acid of the formula (VIII) wherein

$R^{10'}$ denotes hydrogen and the amino group of the aminothiazole ring is protected is obtained from this compound (XIV) by alkaline hydrolysis.

14. Use of compounds of the formula (I) according to Claim 1 for the preparation of medicaments.

**Revendications**

1. Composés de formule (I)

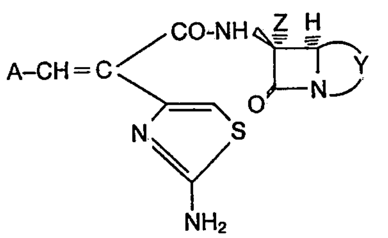

(I)

dans laquelle

A représente l'hydrogène, un groupe alkyle, alcényle ou alcynyle éventuellement cyclique contenant jusqu'à 18 atomes de carbone, qui peut être substitué par O, S ou un groupe méthoxyimino, un reste de formule

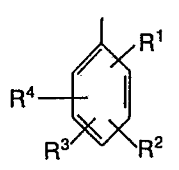

,

dans lequel

$R^1$, $R^2$, $R^3$ et $R^4$ représentent chacun, indépendamment l'un de l'autre, l'hydrogène, un halogène, un groupe alkyle contenant jusqu'à 6 atomes de carbone, un groupe aryle, un groupe

$-OCOR^5$, un groupe 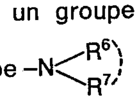 ,

nitro, cyano, trifluorométhyle, hydroxy, alcoxy ou alkylthio contenant jusqu'à 6 atomes de carbone, hydroxycarbonyle, alcoxycarbonyle contenant jusqu'à 6 atomes de carbone dans la partie alkyle, aminocarbonyloxy, sulfonyle ou sulfo,

un groupe aryle étant un cycle aromatique homogène substitué ou non ou un noyau hétérocyclique à 5 ou 6 chaînons,

$R^5$ représente un groupe alkyle, alcényle ou alcynyle éventuellement substitué et éventuellement cyclique contenant jusqu'à 6 atomes de carbone,

$R^6$ et $R^7$ représentent chacun, indépendamment l'un de l'autre, l'hydrogène, un groupe alkyle, alcényle ou alcynyle éventuellement substitué et éventuellement cyclique contenant jusqu'à 6 atomes de carbone ou un groupe alcanoyle contenant jusqu'à 6 atomes de carbone, ou bien, ensemble, un groupe alkyle ou alcanoyle contenant jusqu'à 6 atomes de carbone, un groupe naphtyle, 5-méthylisoxazole-3-yle, furyle, thyényle ou pyridinyle,

Y, à l'état d'acide libre, représente un reste de formule

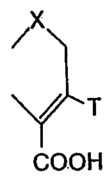

ou

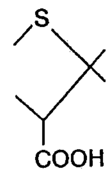

dans lequel

X représente le soufre ou l'oxygène et

T un reste usuel dans la chimie des céphalosporines, et

Z représente H ou un groupe méthoxy.

2. Composés selon la revendication 1, caractérisés en ce que

T représente l'hydrogène, un groupe alkyle en $C_1$–$C_4$, un halogène, un groupe alcoxy en $C_1$–$C_4$, hydroxyméthyle, formyloxyméthyle, (alkyle en $C_1$–$C_4$)-carbonyloxyméthyle, aminocarbonyloxyméthyle, pyridiniumméthyle, 4-carbamoylpyridinium-méthyle ou hétérocyclylthiométhyle, le groupe hétérocyclyle étant un groupe de formule

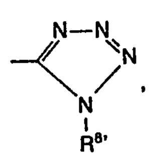 , 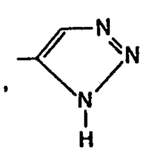 , 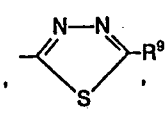 , 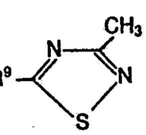

dans lequel

$R^8$ représente l'hydrogène, un groupe méthyle, 2-diméthylaminoéthyle, carboxyméthyle ou sulfométhyle et

$R^9$ représente l'hydrogène ou un groupe méthyle.

3. Composés selon les revendications 1 et 2, caractérisés en ce que

Z représente l'hydrogène ou un groupe méthoxy,

X représente le soufre,

T représente un groupe

$-CH_2OCOCH_3$, 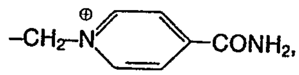 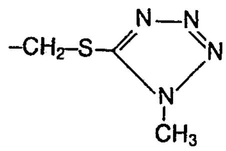,

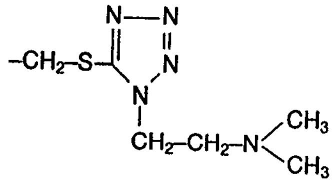 ou $-CH_2OCONH_2$

et

A représente un groupe de formule

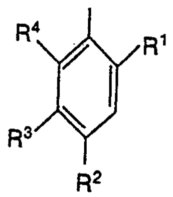, 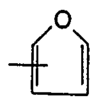, 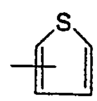, 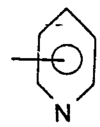, 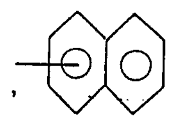

dans lequel les symboles

$R^1$ à $R^4$ ont les significations indiquées dans la revendication 1.

4. Sels acceptables pour l'usage pharmaceutique des composés des revendications 1 à 3.

5. Composés selon l'une des revendications 1 à 4, en configuration Z.

6. Procédé de préparation des composés de formule (I)

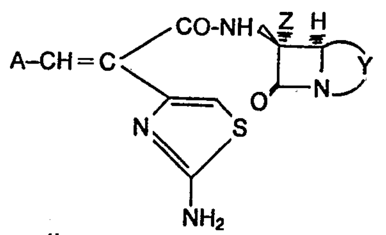 (I)

dans laquelle

A représente l'hydrogène, un groupe alkyle, alcényle ou alcynyle éventuellement cyclique contenant jusqu'à 18 atomes de carbone, qui peut être substitué par O, S ou un groupe méthoxyimino, un reste de formule

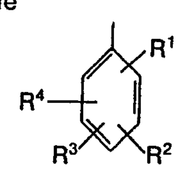,

dans lequel

$R^1$, $R^2$, $R^3$ et $R^4$ représentent chacun, indépendamment l'un de l'autre, l'hydrogène, un halogène, un groupe alkyle contenant jusqu'à 6 atomes de carbone, un groupe aryle, un groupe

$-OCOR^5$, un groupe 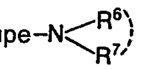,

nitro, cyano, trifluorométhyle, hydroxy, alcoxy ou alkylthio contenant jusqu'à 6 atomes de carbone, hydroxycarbonyle, alcoxycarbonyle contenant jusqu'à 6 atomes de carbone dans la partie alkyle, aminocarbonyloxy, sulfonyle ou sulfo,

un groupe aryle étant un cycle aromatique homogène substitué ou non ou un noyau hétérocyclique à 5 ou 6 chaînons,

$R^5$ représente un groupe alkyle, alcényle ou alcynyle éventuellement substitué et éventuellement cyclique contenant jusqu'à 6 atomes de carbone,

$R^6$ et $R^7$ représentent chacun, indépendamment l'un de l'autre, l'hydrogène, un groupe alkyle, alcényle ou alcynyle éventuellement substitué et eventuellement cyclique contenant jusqu'à 6 atomes de carbone ou un groupe alcanoyle contenant jusqu'à 6 atomes de carbone ou bien, ensemble, un groupe alkyle ou alcanoyle contenant jusqu'à 6 atomes de carbone, une groupe naphtyle, 5-méthylisoxazole-3-yle, furyle, thiényle ou pyridinyle,

Y, à l'état d'acide libre, représente un reste de formule

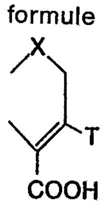 ou 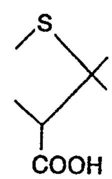

dans lequel

X représente le soufre ou l'oxygène et

T un reste usuel dans la chimie des céphalosporines, et

Z représente H ou un groupe méthoxy, caractérisé en ce que l'on fait réagir des composés de formule générale (II)

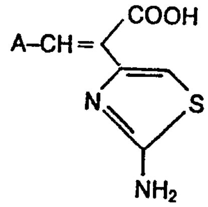 II

dans laquelle

A a les significations indiquées ci-dessus, dans lesquels le groupe amino peut être protégé ou

non, après activation du groupe carboxyle par conversion en un anhydride mixte ou après conversion en l'halogénure d'acide ou après conversion en un ester activé, avec des composés de formule générale (III) éventuellement à l'état mono- ou di-silylé ou à l'état d'esters scindables

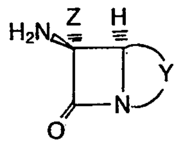

III,

dans lesquels

Y et Z ont les significations indiquées ci-dessus, après quoi, le cas échéant, on élimine les groupes protecteurs.

7. Procédé selon la revendication 6, caractérisé en ce que l'on fait réagir des composés de formule (II), après activation du groupe carboxyle par le pentachlorure de phosphore ou l'oxychlorure de phosphore ou à l'état d'ester d'un alcool de formule (IV)

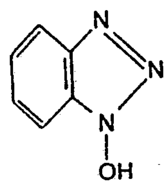

IV,

sans introduction préalable d'un groupe protecteur du groupe amino, avec un composé de formule (III).

8. Procédé de préparation des composés de formule (I), caractérisé en ce que l'on fait réagir des composés de formule (I) selon la revendication 6, dans lesquels T représente un groupe $-CH_2-OCO$-alkyle en $C_1-C_4$ avec des agents nucléophiles, en particulier

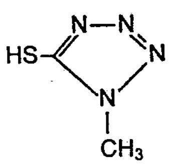

ou

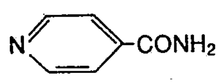

9. Procédé de préparation des composés de formule (I), caractérisé en ce que l'on fait réagir des composés de formule (I) selon la revendication 6, dans lesquels T représente $-CH_2OH$, avec $O=C=NSO_2Cl$ ou $O=C=N-COCCl_3$ et on élimine ensuite le groupe $-SO_2Cl$ ou $-COCCl_3$.

10. Composés de formule générale

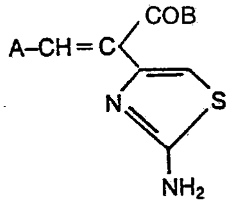

dans laquelle

A a les significations indiquées dans la revendication 1 et

B représente un groupe OH, un halogène, un groupement activant les groupes carboxyle ou un groupe alcoxy en $C_1-C_4$.

11. Procédé de préparation des composés de formule (VIII)

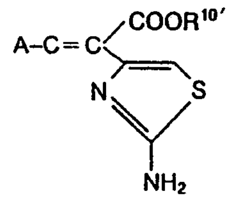

VIII,

dans laquelle

$R^{10'}$ représente un groupe alkyle en $C_1-C_4$ ou H et

A a les significations indiquées dans la revendication 6,

caractérisé en ce que, dans un premier stade opératoire, on prépare un composé de formule (V)

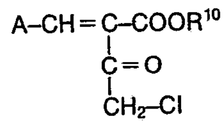

V,

dans laquelle

A a les significations indiquées ci-dessus et

$R^{10}$ représente un groupe alkyle inférieure en $C_1-C_4$, en particulier un groupe éthyle, par condensation d'un composé de formule (VI)

$$Cl-CH_2-CO-CH_2-COOR^{10}, \quad (VI)$$

dans laquelle

$R^{10}$ a les significations indiquées ci-dessus, avec un composé carbonylé de formule (VII)

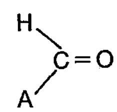

VII,

dans laquelle

A a les significations indiquées ci-dessus, puis on fait réagir le composé de formule (V) avec la thiourée et on saponifie éventuellement un composé de formule (VIII) en l'acide carboxylique libre.

12. Procédé pour la préparation des composés de formule (VIII) selon la revendication 11, dans laquelle

$R^{10'}$ représente un groupe éthyle, caractérisé en ce que l'on fait réagir le produit de condensation IX de l'acétylacétate d'éthyle et d'un composé carbonylé de formule (VII) avec le brome, ce qui donne un 1,4-dibromure X selon le schéma suivant:

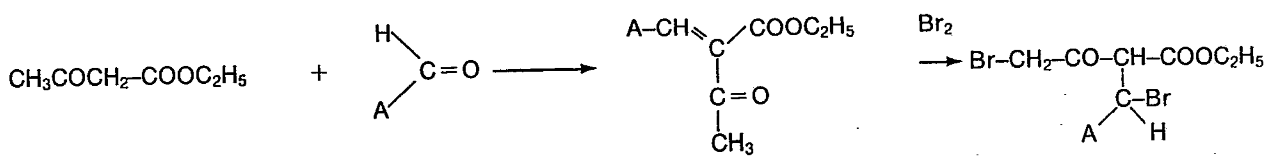


VII IX X,

après quoi on fait réagir le composé de formule (X) avec la thiourée, avec simultanément cyclisation et élimination d'HBr.

13. Procédé de préparation des composés de formule (VIII) selon la revendication 11, dans laquelle

$R^{10'}$ représente l'hydrogène et le groupe amino du cycle aminothiazole est protégé, caractérisé en ce que l'on fait réagir les composés de formule générale (XII)

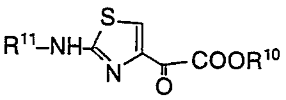

XII

dans laquelle

$R^{10}$ a les significations indiquées dans la revendication 11 et

$R^{11}$ représente un groupe protecteur du groupe amino, par exemple formyle ou tert-butoxycarbonyle,

avec un phosphonate de formule générale (XIII)

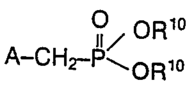

XIII

dans laquelle

A et $R^{10}$ ont les significations indiquées dans la revendication 11,

après déprotonation de (XIII) à l'aide d'une base telle que l'hydrure de sodium, le diisopropylamidure de lithium, l'hydrure de lithium, le butyllithium ou le tert-butanolate de potassium, dans un solvant organique tel que le tétrahydrofuranne, ce qui donne un composé de formule générale (XIV)

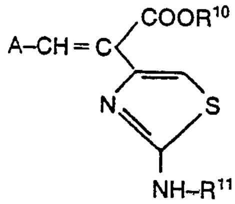

XIV

à partir duquel, par saponification alcaline, on obtient un acide carboxylique de formule (VIII) dans laquelle

$R^{10'}$ représente l'hydrogène et le groupe amino du cycle aminothiazole est protégé.

14. Utilisation des composés de formule (I) selon la revendication 1 pour la préparation de médicaments.